(19) Europäisches Patentamt
European Patent Office
Office européen des brevets

(11) **EP 4 741 832 A1**

(12) **DEMANDE DE BREVET EUROPEEN**

(43) Date de publication:
**13.05.2026 Bulletin 2026/20**

(21) Numéro de dépôt: **25214081.9**

(22) Date de dépôt: **06.11.2025**

(51) Classification Internationale des Brevets (IPC):
**G01P 5/12** *(2006.01)* **A61B 5/087** *(2006.01)*
**G01P 1/00** *(2006.01)* **A61B 5/097** *(2006.01)*
**A61B 5/00** *(2006.01)*

(52) Classification Coopérative des Brevets (CPC):
**A61B 5/0878; A61B 5/097; A61B 5/6819;
A61B 5/682; G01P 5/12;** A61B 2560/0425;
A61B 2560/0431; A61B 2560/0462

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**
Etats d'extension désignés:
**BA**
Etats de validation désignés:
**GE KH LA MA MD TN**

(30) Priorité: **08.11.2024 FR 2412274**

(71) Demandeur: **Aerophonoscope**
**22210 Plemet (FR)**

(72) Inventeurs:
• **GUILLERM, Gérard**
**67260 Diedendorf (FR)**
• **COLLARDEAU, David**
**Ras Hong-Kong (CN)**

(74) Mandataire: **Regimbeau**
**20, rue de Chazelles**
**75847 Paris Cedex 17 (FR)**

(54) **APPAREIL DE MESURE DE RESPIRATIONS BUCCALES ET NASALES, ET PROCÉDÉS DE COMMANDE ET D'ASSEMBLAGE D'UN TEL APPAREIL DE MESURE**

(57) Appareil de mesure de respirations buccales et nasales d'un utilisateur, comprenant une sonde (2) délimitant intérieurement trois canaux de passage d'air avec des ouvertures respectives (22a, 22b, 23), la sonde (2) comprenant une carte électronique (6) comprenant un microcontrôleur (61) et une pluralité de circuits de mesure, comprenant chacun un capteur de mesure de flux d'air (4a, 4b) agencé dans le canal de passage d'air, et un amplificateur, le microcontrôleur (61) étant en outre configuré pour :
- mesurer une tension amplifiée en sortie de l'amplificateur,
- calculer une amplitude de la mesure de tension amplifiée,
- modifier une tension de référence fournie à l'amplificateur pour maintenir l'amplitude constante, et
- estimer une grandeur représentative du flux d'air.

[Fig. 4]

# Description

## DOMAINE TECHNIQUE

La présente invention se rapporte au domaine général des appareils de mesure de de respirations buccales et nasales d'un utilisateur. L'invention s'applique ainsi à un appareil de mesure de respirations buccales et nasales d'un utilisateur, ainsi qu'à un procédé de commande d'un circuit de mesure d'un tel appareil de mesure et à un procédé d'assemblage d'un tel appareil de mesure.

## ETAT DE LA TECHNIQUE

Dans le cadre de l'orthophonie, l'évaluation des modes respiratoires, qu'ils soient buccaux ou nasaux, joue un rôle crucial pour identifier et traiter les dysfonctionnements oro-faciaux, comme des troubles des voies respiratoires ou une insuffisance vélo-pharyngée. Les appareils de mesure des respirations buccales et nasales permettent de quantifier le flux d'air en analysant par exemple la fréquence, la durée et la qualité de la respiration d'un utilisateur. Ils offrent une aide précieuse pour diagnostiquer les habitudes respiratoires anormales, souvent liées à des pathologies telles que l'apnée du sommeil, les obstructions nasales, ou encore les malformations oro-faciales, et facilitent ainsi la mise en place d'interventions ciblées en rééducation. Leur utilisation permet aux orthophonistes de suivre l'évolution de la respiration d'un utilisateur tel qu'un patient et d'adapter les thérapies en conséquence, améliorant ainsi la qualité de vie des personnes affectées.

S'il existe des méthodes de diagnostic invasives comme la naso-endoscopie, des appareils non invasifs tels que le rhinomanomètre, le spiromètre ou le pharyngomètre acoustique ont également été développés et permettent chacun des diagnostics spécifiques. Par exemple, le rhinomanomètre permet d'évaluer l'efficacité respiratoire nasale et de détecter d'éventuelles obstructions en mesurant la résistance du passage de l'air dans les voies nasales, tandis que le spiromètre permet d'évaluer les capacités respiratoires en mesurant les volumes d'air inspirés et expirés par les poumons.

Plus récemment, le document WO 2016/128627 A1 a proposé un appareil permettant de détecter à la fois les respirations buccales et nasales d'un patient, afin de pouvoir simultanément évaluer les capacités fonctionnelles du voile du palais et détecter des anomalies respiratoires, et d'analyser les effets de la rééducation orthophonique ou des améliorations suites à une expansion maxillaire par exemple.

Toutefois, l'appareil proposé permet de détecter un écoulement d'air mais ne permet pas d'obtenir des mesures suffisamment précises du débit du flux d'air alternatif lors de la respiration du patient. De plus, il existe un besoin d'améliorer la fiabilité d'un tel appareil. En effet, la qualité de mesure se dégrade rapidement lorsque la durée d'utilisation de l'appareil augmente.

## EXPOSE DE L'INVENTION

Un but de la présente invention est de proposer un appareil de mesure de respiration d'un utilisateur ayant une précision satisfaisante tout en présentant une meilleure fiabilité de la mesure dans le temps.

A cet effet, l'invention propose, selon un premier aspect, un appareil de mesure de respirations buccales et nasales d'un utilisateur, comprenant une sonde délimitant intérieurement

un premier canal de passage d'air comprenant une première ouverture, la première ouverture étant propre à être positionnée sous une première narine de l'utilisateur,
un deuxième canal de passage d'air comprenant une deuxième ouverture, la deuxième ouverture étant propre à être positionnée sous une deuxième narine de l'utilisateur, et
un troisième canal de passage d'air comprenant une troisième ouverture, la troisième ouverture étant propre à être positionnée en vis-à-vis de la bouche de l'utilisateur,

la sonde comprenant
une carte électronique comprenant un microcontrôleur et une pluralité de circuits de mesure, chaque circuit de mesure étant propre à un canal de passage d'air associé, l'appareil de mesure étant remarquable en ce que chaque circuit de mesure comprend

un capteur de mesure de flux d'air agencé dans le canal de passage d'air associé et comprenant un filament métallique, autour duquel est propre à s'écouler un flux d'air s'écoulant dans le canal passage d'air associé, chaque capteur de mesure de flux d'air étant propre à être alimenté par un courant électrique lors d'une respiration de l'utilisateur à travers l'appareil, et
un amplificateur configuré pour fournir en sortie une tension amplifiée à partir d'une tension de référence et d'une tension aux bornes du filament métallique du capteur de mesure de flux d'air alimenté,

le microcontrôleur étant en outre configuré pour :

- mesurer la tension amplifiée en sortie de l'amplificateur,
- calculer une amplitude de la mesure de tension amplifiée,
- modifier la tension de référence en fonction de la mesure de tension amplifiée, de sorte à maintenir l'amplitude constante, et

- estimer une grandeur représentative du flux d'air s'écoulant dans le canal de passage d'air associé au capteur de mesure de flux d'air alimenté à partir de la mesure de tension amplifiée.

Ainsi, le microcontrôleur est configuré pour modifier la tension de référence en entrée de l'amplificateur de manière dynamique, pour réaliser un asservissement de l'amplitude de la mesure de tension amplifiée. Cela permet que le capteur actif, soumis à un flux d'air de respiration circulant dans le canal de passage d'air où il est disposé, présente une sensibilité constante au cours du temps. En particulier, cela empêche une dérive de l'amplitude de la mesure lors de variations des propriétés physiques du filament chauffé du capteur est alimenté en courant électrique, typiquement sa résistance.

L'appareil de mesure selon l'invention est avantageusement complété par les caractéristiques suivantes, prises individuellement ou en l'une quelconque de leurs combinaisons techniquement possibles :

- l'amplitude de signal est calculée selon un cycle comprenant une période, la période dépendant de l'estimée de la grandeur représentative. Cela permet d'actualiser la valeur de l'amplitude de signal, notamment à chaque changement de direction du flux d'air correspondant à un instant où la vitesse du flux d'air est nul, entre une inspiration et une expiration ;
- chaque circuit de mesure comprend en outre une source de courant configurée pour délivrer un courant électrique de sorte à alimenter le filament métallique du capteur de mesure de flux d'air, le courant électrique délivré ayant une intensité constante inférieure à 100mA. Typiquement, la source de courant est un puits de courant. Cela permet d'avoir une faible consommation électrique de l'appareil de mesure, et donc une meilleure autonomie lorsque l'appareil de mesure est alimenté par une batterie ;
- le microcontrôleur est en outre configuré pour commander un préchauffage du filament métallique d'au moins un capteur de mesure de flux d'air pendant une durée prédéterminée avant de mesurer la tension amplifiée. Le préchauffage permet de réduire les variabilités d'estimation de la grandeur représentative du flux due à la variation de la résistance du filament métallique lors de l'augmentation rapide en température du filament métallique au début de son alimentation ;
- le microcontrôleur est en outre configuré pour modifier une intensité du courant électrique délivré à partir de la mesure de tension amplifiée. Cela permet notamment un calibrage de l'appareil de mesure au moment du préchauffage, et d'augmenter la fiabilité de la mesure ;
- la sonde comprend en outre un capteur de température configuré pour fournir une mesure de température au microcontrôleur. Cela permet de prendre en compte l'évolution de la température ambiante ou du flux d'air dans l'estimation de la grandeur représentative du flux d'air, et donc la fiabilité de la mesure ;
- le microcontrôleur comprend en outre une horloge configurée pour mesurer une durée d'alimentation du filament métallique du capteur de mesure de flux d'air alimenté, et le microcontrôleur est configuré pour émettre un signal de maintenance lorsque la durée d'alimentation mesurée dépasse une durée maximale d'utilisation. Cela permet d'éviter d'utiliser l'appareil de mesure lorsque le risque d'avoir une mesure de faible qualité ou non fiable est important ;
- les capteurs de mesure de flux d'air sont assemblés sur un circuit imprimé modulaire connecté à la carte électronique de manière amovible. Cela permet de simplement et rapidement changer les capteurs de mesure de flux d'air, lorsque la qualité de la mesure est compromise due à l'usure du filament métallique.
- l'au moins un capteur de mesure de flux d'air comprend un substrat, de préférence en céramique. Cela permet d'améliorer la résistance du capteur et donc d'augmenter la durée de vie de l'appareil de mesure.
- le filament métallique comprend une pluralité de pistes résistives intégrées dans le substrat ou le filament métallique est formé par impression ou revêtement d'un matériau métallique électriquement conducteur sur le substrat, le matériau métallique électriquement conducteur étant de préférence du platine. Cela permet d'avoir un procédé de fabrication du capteur simple et maîtrisé à l'échelle industrielle. L'utilisation de tels filaments métalliques permet d'avoir une mesure précise et de réduire la consommation électrique du capteur, et ainsi d'augmenter l'autonomie de l'appareil de mesure.
- le substrat et/ou le filament métallique sont recouverts par une couche de protection. Cela permet d'augmenter encore la résistance du capteur et sa plage de fonctionnement (notamment en température), et donc d'augmenter la durée de vie de l'appareil de mesure.
- le substrat est une plaque formant une grille. Cela permet au flux d'air de circuler autour du filament métallique, tout en permettant au filament métallique d'être supporté par le substrat avec une certaine flexibilité.
- le substrat est souple, de sorte que le substrat est propre à se déformer élastiquement lorsque le flux d'air s'écoule à travers le substrat et autour du filament métallique. Cela permet de faciliter l'intégration du capteur à l'appareil de mesure et d'autoriser des déformations réversibles du capteurs lors du passage du flux d'air, et donc d'augmenter la durée de vie de l'appareil de mesure.

Selon un deuxième aspect, l'invention propose un procédé de commande d'un circuit de mesure d'un appareil de mesure tel que décrit précédemment, mis en œuvre par un microcontrôleur, le circuit de mesure comprenant un amplificateur configuré pour fournir en sortie une tension amplifiée à partir d'une tension de référence et d'une tension aux bornes d'un filament métallique d'un capteur actif, le capteur actif étant agencé dans un canal de passage d'air, le procédé de commande comprenant les étapes de :

- mesure d'une tension amplifiée en sortie de l'amplificateur,
- calcul d'une amplitude de signal à partir de la mesure de tension amplifiée,
- modification de la tension de référence en fonction de la mesure de tension amplifiée, de sorte à maintenir l'amplitude de signal constante,
- estimation d'une grandeur représentative d'un flux d'air s'écoulant dans le canal de passage d'air à partir de la mesure de tension amplifiée.

Selon un troisième aspect, l'invention propose un procédé d'assemblage d'un appareil de mesure tel que décrit précédemment, comprenant les étapes suivantes :

- assemblage d'une pluralité d'ampoules à incandescence sur un circuit imprimé, chaque ampoule à incandescence comprenant un bulbe de protection recouvrant un filament métallique,
- retrait du bulbe de protection de chaque ampoule à incandescence de sorte à obtenir une pluralité de capteurs de mesures de flux d'air,
- optionnellement, pliage d'au moins un capteur de mesure de flux d'air, et
- intégration du circuit imprimé portant la pluralité de capteurs de mesures de flux d'air sur une carte électronique de la sonde.

Un tel procédé permet de simplement et rapidement changer les capteurs de mesure de flux d'air, en limitant le risque de rupture du filament métallique, lorsque la qualité de la mesure est compromise due à l'usure du filament métallique d'un des capteurs de mesure de flux d'air. Cela permet aussi d'utiliser comme capteurs des ampoules à incandescence facilement accessibles dans le commerce et peu onéreuses.

**DESCRIPTION DES FIGURES**

D'autres caractéristiques, buts et avantages de l'invention ressortiront de la description qui suit, qui est purement illustrative et non limitative, et qui doit être lue en regard des dessins annexés, donnés à titre d'exemples non limitatifs et sur lesquels :

La figure 1 est une vue en perspective d'un appareil de mesure selon un mode de réalisation.

La figure 2 est une vue de dessus de l'appareil de mesure de la figure 1.

La figure 3 est une section de l'appareil de mesure de la figure 1 dans un plan facial.

La figure 4 est une vue en perspective d'une coupe dans le plan facial de l'appareil de mesure de la figure 1.

La figure 5 est une section de l'appareil de mesure de la figure 1 dans un plan latéral.

La figure 6 est une représentation schématique d'un capteur de mesure de flux d'air de l'appareil dans un premier mode de réalisation.

La figure 7 est une représentation schématique d'un capteur de mesure de flux d'air de l'appareil dans un deuxième mode de réalisation.

La figure 8 est une représentation schématique d'un capteur de mesure de flux d'air de l'appareil dans un troisième mode de réalisation.

La figure 9 est une représentation schématique du circuit de mesure de la carte électronique de l'appareil de mesure.

La figure 10 est un organigramme des étapes d'un procédé de commande d'un circuit de mesure de l'appareil de mesure.

La figure 11 est une représentation schématique d'une unité de traitement pour l'analyse de mesures de respiration de l'appareil de mesure.

La figure 12 est un organigramme des étapes d'un procédé d'assemblage du circuit de mesure de l'appareil de mesure.

La figure 13 est une vue en perspective d'un circuit imprimé modulaire au cours d'une étape d'assemblage des capteurs dans le premier mode de réalisation.

La figure 14 illustre trois vues en perspective du circuit imprimé modulaire au cours d'une étape de découpage des capteurs dans le premier mode de réalisation.

La figure 15 illustre deux vues en perspective du circuit imprimé modulaire au cours d'une étape de pliage d'un des capteurs dans le premier mode de réalisation.

La figure 16 est une vue en perspective du circuit imprimé modulaire au cours d'une étape de protection des capteurs dans le premier mode de réalisation.

La figure 17 est une vue en perspective de la sonde de l'appareil lors d'une étape d'intégration du circuit imprimé modulaire du procédé d'assemblage.

La figure 18 est une section de l'appareil de mesure dans un plan facial, dans les deuxième et troisième modes de réalisation des capteurs.

La figure 19 est une vue en perspective d'une coupe de l'appareil de mesure sur lequel des capteurs selon le deuxième mode de réalisation sont assemblés.

La figure 20 illustre trois vues en perspective de la sonde de l'appareil au cours d'un procédé de maintenance des capteurs.

Sur l'ensemble des figures, les éléments similaires portent des références identiques.

## DESCRIPTION DETAILLEE DE L'INVENTION

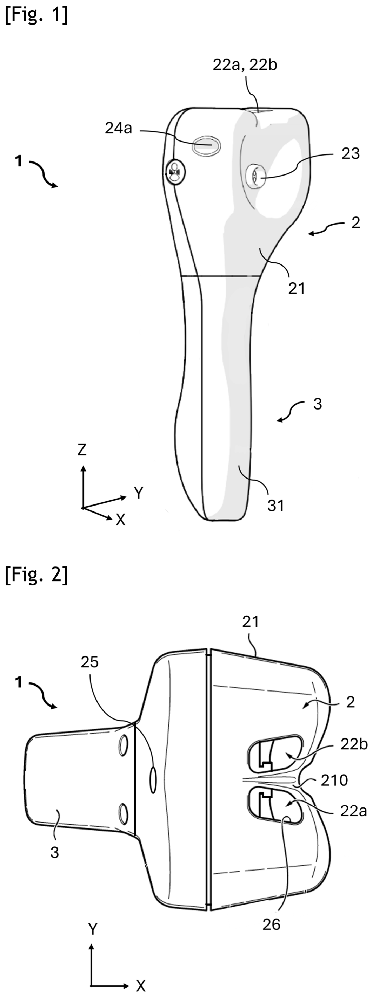

En référence aux figures 1 à 5, on considère un appareil de mesure 1 de la respiration buccale et de la respiration nasale d'un utilisateur. L'utilisateur peut être un patient dans le cadre d'un diagnostic médical ou du suivi d'une rééducation, mais l'appareil de mesure proposé peut également être utilisé dans un contexte non médical, par exemple de loisir ou de jeu.

*Structure de l'appareil*

L'appareil de mesure 1 de respirations tel que décrit comprend une sonde 2 propre à être agencée dans une position de mesure en vis-à-vis du visage de l'utilisateur pour la mesure de ses respirations. La sonde 2 forme une unité de mesure comprenant une pluralité de capteurs de mesure de respiration, comme il sera détaillé par la suite.

Dans ce mode de réalisation, l'appareil de mesure 1 comprend une poignée 3 propre à être saisie par l'utilisateur pour positionner la sonde 2 dans la position de mesure. De manière alternative, la sonde 2 peut être intégrée dans un support propre à être positionné sur l'utilisateur, par exemple un casque, ou encore être maintenue dans la position de mesure par un système de maintien, par exemple des sangles ou des élastiques.

En référence à la figure 1, la poignée 3 est formée par une paroi externe 31 servant à la préhension par la main de l'utilisateur, dont les narines sont positionnées en face d'au moins une ouverture 22a, 22b disposée en partie supérieure de la sonde 2, de préférence d'une première ouverture 22a et d'une deuxième ouverture 22b, et dont la bouche est positionnée en face d'une troisième ouverture 23, laquelle est disposée en partie inférieure de la sonde 2. La poignée 3 s'étend selon une direction longitudinale Z. La forme de la poignée 3 est choisie afin de permettre une saisie à une main de l'appareil de mesure 1 de manière ergonomique.

De préférence, la sonde 2 est assemblée sur la poignée 3 de manière amovible. En d'autres termes, la sonde 2 est fixée réversiblement sur la poignée 3. La sonde 2 est alors fixée à la poignée 3 dans une position de solidarisation de manière réversible, c'est-à-dire qu'elle peut être montée sur la poignée 3 ou retirée de la poignée 3 par l'utilisateur ou par un opérateur. Cela permet d'effectuer simplement une maintenance en cas de panne ou de défaillance d'un des capteurs de mesure de respiration présents dans la sonde 2.

De préférence, l'appareil de mesure 1 comprend plusieurs sondes 2 amovibles, pouvant être montées chacune d'une manière interchangeable dans la position de solidarisation sur la poignée 3. Cela permet d'utiliser des sondes 2 de tailles différentes permettent de s'adapter à la morphologie ou à l'âge de l'utilisateur. Cela permet également d'utiliser une sonde 2 différente selon l'usage de l'appareil de mesure 1, par exemple une sonde 2 pour un diagnostic d'orthodontie, une sonde 2 pour un diagnostic d'orthophonie ou une sonde 2 pour un usage de loisir.

Cela permet également d'assurer la compatibilité de sondes futures et donc d'améliorer l'appareil de mesure 1 en remplaçant la sonde 2 ou la poignée 3 par des modèles plus récents.

La sonde 2 comprend une enveloppe extérieure formée par une coque 21. Dans le mode de réalisation représentée, la coque 21 comprend une première coque formant une paroi avant de la sonde 2 de l'appareil de mesure 1, et une deuxième coque formant une paroi arrière de la sonde 2.

La coque 21 comprend une paroi supérieure présentant au moins une ouverture, de préférence une première ouverture 22a et une deuxième ouverture 22b. L'ouverture, de préférence chacune de la première ouverture 22a et de la deuxième ouverture 22b, comprend au moins un orifice permettant un écoulement d'air à travers la coque 21 lors de la respiration nasale. L'ouverture, ou la première ouverture 22a et la deuxième ouverture 22b, sont ainsi destinées à être positionnées sous les narines de l'utilisateur, au moins lorsque la mesure de respiration nasale est réalisée.

Dans le mode de réalisation illustré, l'appareil de mesure 1 comprend une première ouverture 22a et une deuxième ouverture 22b, dites ouvertures supérieures, chacune correspondant à une narine de l'utilisateur lorsque l'appareil de mesure 1 est disposé dans la position de mesure, c'est-à-dire contre le visage de l'utilisateur. En d'autres termes, la première ouverture 22a est propre à être positionnée sous une première narine de l'utilisateur (ici la narine gauche), et la deuxième ouverture 22b est propre à être positionnée sous une deuxième narine de l'utilisateur (ici la narine droite). On entend par « sous » que l'ouverture est située en regard ou en vis-à-vis de la narine associée, et suffisamment proche de cette dernière pour que l'air expiré par la narine pénètre dans la sonde 2 par l'ouverture correspondante, à savoir la première ouverture 22a ou la deuxième ouverture 22b.

La sonde 2 comprend également une troisième ouverture 23, dite ouverture buccale ou ouverture inférieure, propre à être positionnée en vis-à-vis de la bouche de l'utilisateur. Plus précisément, la coque 21 comprend une paroi avant présentant la troisième ouverture 23. La troisième ouverture 23 comprend au moins un orifice permettant un écoulement d'air à travers la coque 21 lors de la respiration buccale. Dans la position de mesure, la troisième ouverture 23 est ainsi positionnée devant la bouche de l'utilisateur, et suffisamment proche pour que l'air expiré par la bouche pénètre dans la sonde 2 par la troisième ouverture 23.

Ainsi, la première ouverture 22a et la deuxième ouverture 22b correspondent à des entrées d'air d'expiration nasale et des sorties d'air d'inspiration nasale, et la troisième ouverture 23 correspond à une entrée d'air d'expiration buccale et une sortie d'air d'inspiration buccale.

Afin d'améliorer le confort de l'utilisateur, la coque 21 peut comprendre une échancrure 210 s'étendant sur la paroi supérieure de la coque 21 depuis la paroi avant, entre la première ouverture 22a et la deuxième ouverture 22b. L'échancrure 210 est ergonomique et présente une forme adaptée à loger l'arête inférieure du nez de l'utilisateur. Dans la position de mesure, le nez de l'utilisateur est ainsi posé contre la paroi supérieure de la coque 21, chaque narine coïncidant avec une ouverture 22a, 22b associée de part et d'autre de l'échancrure 210.

Typiquement, la paroi avant de la coque 21 comprend un renfoncement ouvert vers l'avant, permettant à l'utilisateur de disposer confortablement sa bouche contre l'appareil de mesure 1 dans la position de mesure. Dans le mode de réalisation représenté, le ou les orifices de la troisième ouverture 23 sont entourés par une collerette afin de permettre un bon positionnement de la lèvre supérieure et/ou inférieure de la bouche au niveau de la troisième ouverture 23. Cela permet également d'améliorer la fiabilité et la qualité de la mesure effectuée avec l'appareil de mesure 1.

La sonde 2 délimite intérieurement un premier canal de passage d'air Ca comprenant la première ouverture 22a, un deuxième canal de passage d'air Cb comprenant la deuxième ouverture 22b, et un troisième canal de passage d'air Cc comprenant la troisième ouverture 23. Par « passage d'air », il est entendu qu'un flux d'air de respiration peut s'écouler dans chaque canal de passage d'air Ca, Cb, Cc de manière bidirectionnelle.

Le premier canal de passage d'air Ca et le deuxième canal de passage d'air Cb peuvent être délimités par la coque 21, c'est-à-dire solidaires de la coque 21, ou être obtenus par assemblage d'une pièce rapportée, par exemple un tuyau flexible. L'étanchéité entre le premier canal de passage d'air Ca et la coque 21 ou entre le deuxième canal de passage d'air Cb et la coque 21 peut être assurée par l'ajout d'un joint d'étanchéité. De même, les capteurs de la sonde 2 sont assemblés dans leur canal de passage d'air respectif en utilisant un joint d'étanchéité décrit par la suite, afin d'assurer qu'aucun air ne circulant dans un canal de passage d'air Ca, Cb, Cc ne s'échappe par un autre orifice que les ouvertures prévues à cet effet.

De préférence, le premier canal de passage d'air Ca et le deuxième canal de passage d'air Cb ne communiquent pas entre eux, c'est-à-dire qu'ils sont fluidiquement séparés. Cela permet que l'appareil de mesure 1 mesure la respiration de chaque narine de l'utilisateur indépendamment, et permet ainsi de détecter une asymétrie de la respiration nasale.

Dans le mode de réalisation représenté, la paroi latérale de la coque 21 comprend une première ouverture latérale 24a en communication de fluide avec la première ouverture 22a par l'intermédiaire du premier canal de passage d'air Ca, et une deuxième ouverture latérale 24b en communication de fluide avec la deuxième ouverture 22b par l'intermédiaire du deuxième canal de passage d'air Cb.

Chaque ouverture latérale 24a, 24b comprend au moins un orifice permettant un écoulement d'air à travers la coque 21, et notamment la sortie d'air lorsque l'utilisateur expire par le nez à travers l'ouverture 22a, 22b du canal de passage d'air Ca, Cb respectif, et l'entrée d'air lorsque l'utilisateur inspire par le nez à travers l'ouverture 22a, 22b.

La coque 21 comprend une paroi arrière, opposée à la paroi avant par rapport à la sonde 2, et présentant une ouverture arrière 25 en communication de fluide avec la troisième ouverture 23 par l'intermédiaire du troisième canal de passage d'air Cc. L'ouverture arrière 25 comprend typiquement une pluralité d'orifices débouchant sur le troisième canal de passage d'air Cc. Le troisième canal de passage d'air Cc permet la circulation d'un flux d'air buccal entre la bouche de l'utilisateur et l'extérieur de la sonde 2. La direction X correspond ici à une direction de flux buccal. Le troisième canal de passage d'air Cc peut être délimité par la coque 21, c'est-à-dire solidaire de la coque 21, ou être obtenu par assemblage d'une pièce rapportée.

De préférence, le troisième canal de passage d'air Cc ne communique ni avec le premier canal de passage d'air Ca, ni avec le deuxième canal de passage d'air Cb, c'est-à-dire que le troisième canal de passage d'air Cc est fluidiquement séparé du premier canal de passage d'air Ca et du deuxième canal de passage d'air Cb. Cela permet que l'appareil de mesure 1 mesure la respiration buccale de l'utilisateur indépendamment de la respiration nasale, et permet ainsi de détecter une asymétrie entre la respiration nasale et la respiration buccale.

La coque 21 délimite un compartiment 26 dans la sonde 2 dans lequel peuvent être agencés des éléments de commande de l'appareil de mesure 1, par exemple une carte électronique 6. La coque 21 peut former un ensemble étanche. Par exemple, les différents capteurs de mesure de flux d'air 4a, 4b, 4c et composants électroniques contenus dans la sonde 2 sont agencés dans le compartiment 26 séparé de l'extérieur de manière étanche.

L'appareil de mesure 1 est conçu pour être stérilisable par une méthode UV (stérilisation par rayonnement ultraviolet) ou par l'utilisation de lingettes désinfectantes. De préférence, l'appareil de mesure 1 est compatible avec des protocoles de stérilisation standard tels que l'autoclave, la stérilisation sous vide par gaz d'éthylène (EtO), ou un procédé de stérilisation par diffusion de vapeur de peroxyde d'hydrogène tel que proposé par Sterrad (marque déposée).

De préférence, l'appareil de mesure 1 comprend une pièce d'hygiène amovible destinée à être fixée contre la paroi avant de la coque 21. La pièce d'hygiène est configurée pour filtrer les flux d'air buccal ou nasal, et ainsi protéger les capteurs de la sonde 2 de l'humidité ou des particules solides provenant de la respiration de l'utilisateur. Dans un contexte médical, cela permet également de ne pas nécessiter une stérilisation de l'appareil de mesure 1 entre chaque utilisateur, et donc, dans un contexte médical, de faire gagner du temps au praticien tout en limitant le risque de transmission de maladie respiratoire entre deux patients successifs. La pièce d'hygiène amovible correspondant à un masque de protection peut être fixée sur la coque 21 par l'intermédiaire des protubérances au niveau de la première ouverture latérale 24a et de la deuxième ouverture latérale 24b.

La sonde 2 comprend un capteur de mesure de flux d'air 4a, 4b, 4c agencé dans chaque canal de passage d'air Ca, Cb, Cc, chaque capteur de mesure de flux d'air 4a, 4b, 4c étant configuré pour mesurer une grandeur représentative d'un flux d'air s'écoulant dans le canal de passage d'air Ca, Cb, Cc dans lequel il est agencé. Le fonctionnement des capteurs de mesure de flux d'air 4a, 4b, 4c sera détaillé par la suite.

*Capteurs de mesure de respiration*

La sonde 2 est configurée pour fournir une mesure de la respiration buccale et/ou nasale de l'utilisateur. Chaque capteur de mesure de flux d'air 4a, 4b, 4c est apte à fournir au moins un signal de mesure de présence de flux d'air, c'est-à-dire est configuré pour fournir un signal de mesure permettant d'estimer une grandeur représentative du flux d'air, par exemple la vitesse du flux d'air, sa direction, ou son débit.

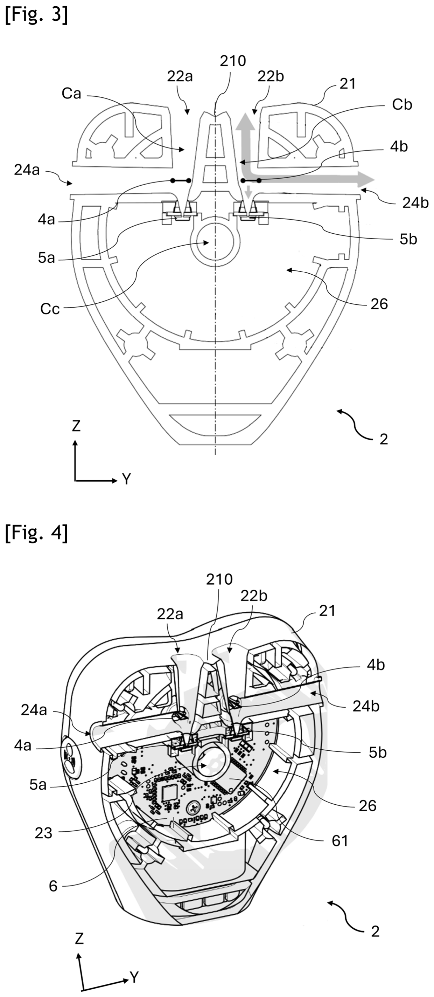

En référence à la vue dans le plan facial (Y, Z) de la figure 3, la sonde 2 présente un axe de symétrie parallèle à la direction longitudinale Z passant par l'échancrure 210. La direction longitudinale Z correspond à une direction de flux nasal.

Le premier canal de passage d'air Ca comprend une première portion ou portion primaire s'étendant depuis la première ouverture 22a vers l'intérieur de la sonde 2 selon la direction Z, et une deuxième portion s'étendant depuis l'ouverture 24a latérale vers l'intérieur de la sonde 2 selon la direction Y. Les deux portions sont séparées par un coude. De manière symétrique, le deuxième canal de passage d'air Cb comprend deux portions s'étendant selon deux directions orthogonales et raccordées par un coude.

La sonde 2 comprend un premier capteur de mesure de flux d'air 4a nasal et un deuxième capteur de flux d'air 4b nasal. Chaque capteur de mesure de flux d'air 4a, 4b est disposé dans un canal de passage d'air Ca, Cb respectif.

Plus précisément, le premier capteur de mesure de flux d'air 4a est disposé au niveau du coude raccordant la portion primaire et la deuxième portion du premier canal de passage d'air Ca. Cela permet que le premier capteur de mesure de flux d'air 4a soit autant exposé au flux d'air s'écoulant depuis la première ouverture 22a lors de l'expiration nasale, ou depuis l'ouverture latérale 24a lors de l'inspiration nasale. De même, le deuxième capteur de mesure de flux d'air 4b est disposé symétriquement dans le coude du deuxième canal de passage d'air Cb.

La sonde 2 comprend également un capteur de mesure de flux d'air 4c buccal disposé dans le troisième canal de passage d'air Cc. Le capteur de mesure de flux d'air 4c buccal est configuré pour mesurer une grandeur relative à l'écoulement d'air buccal entre la troisième ouverture 23 et l'ouverture arrière 25. Le capteur de mesure de flux d'air 4c buccal peut être identique aux capteurs de mesure de flux d'air 4a, 4b nasal. Leur fonctionnement sera détaillé par la suite.

De préférence, la sonde 2 comprend en outre au moins un microphone 5a, 5b, 5c configuré pour détecter une variation de pression telle qu'une surpression dans un des canaux de passage d'air Ca, Cb, Cc lié à un écoulement d'air en provenance de l'ouverture 22a, 22b ou 23 communiquant avec le canal de passage d'air. Cela permet de distinguer simplement une phase d'inspiration d'une phase d'expiration, la surpression activant le microphone 5a, 5b, 5c ne survenant que lors de l'expiration de l'utilisateur. Le microphone 5a, 5b, 5c peut capter des signaux sonores.

Dans le mode de réalisation illustré, l'appareil de mesure 1 comprend avantageusement un premier microphone 5a configuré pour détecter un écoulement d'air en provenance de la première ouverture supérieure 22a, et un deuxième microphone 5b configuré pour détecter un écoulement d'air en provenance de la deuxième ouverture supérieure 22b.

Chacun du premier microphone 5a et du deuxième microphone 5b sont typiquement disposés dans une cavité propre en communication de fluide avec le canal de passage d'air Ca, Cb respectif. Le premier microphone 5a est orienté en vis-à-vis de la première ouverture 22a, et le deuxième microphone 5b est orienté en vis-à-vis de la deuxième ouverture 22b associée. Plus précisément, la cavité propre est disposée dans le prolongement de la première portion du canal de passage d'air Ca, Cb, selon la direction Z d'expiration nasale.

La sonde 2 peut également comprendre un troisième microphone 5c ou microphone buccal 5c configuré pour détecter un écoulement d'air en provenance de la troisième ouverture 23. Le troisième microphone 5c est agencé dans une cavité propre en communication avec le troisième canal de passage d'air Cc, et typiquement orienté en vis-à-vis, c'est-à-dire de sorte à faire face à, la troisième ouverture 23. Le troisième microphone 5c peut être identique au premier microphone 5a et au deuxième microphone 5b. Le troisième microphone 5c est destiné à être situé près de la bouche de l'utilisateur et est particulièrement utile pour détecter les phonèmes.

Ainsi, chaque canal de passage d'air Ca, Cb, Cc peut être associé à un microphone 5a, 5b, 5c configuré pour détecter une variation de pression d'air dans le canal de passage d'air Ca, Cb, Cc associé. Le premier microphone 5a, le deuxième microphone 5b, et le troisième microphone 5c peuvent être de différent types, par exemple de type mécanique, électrostatique, EMC ou MEMS. Le microphone 5a, 5b, 5c a un fort rapport signal sur bruit (SNR pour « Signal-to-Noise Ratio »), typiquement supérieur à 60 dB(A). Par exemple, un microphone de type piézolélectrique n'est pas assez sensible pour détecter une variation de pression d'air.

De préférence, la sonde 2 est en outre configurée pour être connectée à un capteur de vibrations du larynx. Le capteur de vibrations du larynx peut être un capteur externe générique ou embarqué dans un autre appareil, typiquement intégré à une montre connectée. Le capteur de vibrations du larynx peut être de type vibratoire (comme un capteur piézoélectrique ou un accéléromètre), ou encore, de manière non limitative, un capteur de flux sanguins. La coque 21 comprend par exemple un connecteur, typiquement un port jack, relié au circuit électrique 6 et destiné à recevoir un câble jack relié au capteur de vibrations du larynx. De préférence, le capteur de vibrations du larynx est configuré pour échanger des informations avec la carte électronique 6 sans liaison filaire, par exemple via Bluetooth (marque déposée) ou Wi-Fi (marque déposée). Cela permet d'augmenter le confort de l'utilisateur et de faciliter l'utilisation de l'appareil de mesure 1.

Le capteur de vibrations du larynx est destiné à être appliqué sur la gorge de l'utilisateur, au niveau du larynx. Il est maintenu en position par un moyen de fixation adapté, par exemple un adhésif ou un élastique. Le capteur de vibrations du larynx est typiquement un capteur piézoélectrique configuré pour transformer une force provoquée par les vibrations du larynx de l'utilisateur en une tension électrique transmise à la carte électronique 6. Un tel canal d'analyse externe est très utile en orthophonie, car il permet de détecter des sons supplémentaires et une respiration basse, au niveau des poumons. La prononciation du son « A » par exemple n'émet pas de flux d'air, et peut être détectée par le capteur de vibrations du larynx. Le troisième microphone 5c peut être avantageusement utilisé pour détecter des sons émis par l'utilisateur.

La sonde 2 peut comprendre un module d'analyse linguistique configuré pour détecter des mots prononcés et identifier la langue de l'utilisateur. Un tel module d'analyse linguistique peut mettre en œuvre de l'intelligence artificielle. Il peut être implémenté sur le microcontrôleur 61 décrit par la suite, ou sur un microcontrôleur indépendant relié à la carte électronique 6 de la sonde 2. De manière alternative ou complémentaire, le module d'analyse linguistique peut être implémenté dans une unité de traitement 100 à laquelle l'appareil de mesure 1 est connecté.

*Capteurs de mesure de flux d'air*

Par la suite, on désignera par « capteur de mesure de flux d'air 4 » l'un quelconque parmi les capteurs de mesure de flux d'air 4a, 4b nasal et 4c buccal. Ainsi, chacun des capteurs de mesure de flux d'air 4a, 4b nasal et 4c buccal peut être réalisé conformément au capteur de mesure de flux d'air 4.

Le capteur de mesure de flux d'air 4 est de type thermistance. Il est positionné dans le canal de passage d'air Ca, Cb, Cc respectif pour être exposé au flux d'air provenant de ou allant vers l'ouverture 22a, 22b ou 23 associée.

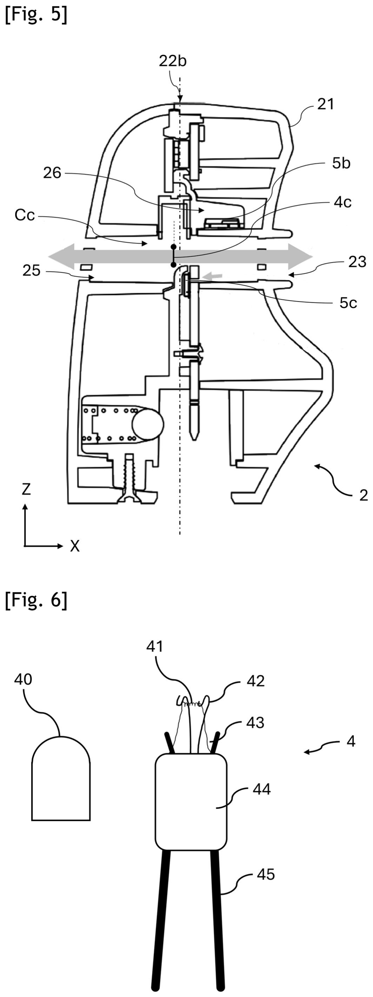

Dans un premier mode de réalisation illustré à la figure 6, le capteur de mesure de flux d'air 4 comprend un filament métallique 41 qui est un conducteur électrique nu de thermistance incandescent, c'est-à-dire que le courant électrique passant par le filament métallique 41 induit une augmentation de la température du filament métallique 41 induisant une émission d'ondes électromagnétiques et de la chaleur. Le conducteur électrique étant nu, une combustion pourrait avoir lieu par l'apport d'oxygène, par exemple en raison de l'oxygène contenu dans l'air. L'intensité du courant traversant le filament métallique 41 est limitée afin d'éviter une consommation rapide du filament métallique 41.

Par exemple, le capteur de mesure de flux d'air 4 représenté correspond à une ampoule à incandescence standard commercialisée par *JKL components corporation* sous la référence 7373 à laquelle on a préalablement retiré le bulbe de protection 40 en verre lors d'un procédé d'assemblage détaillé par la suite. Cela permet que le filament métallique 41 soit en contact direct du flux d'air s'écoulant dans le canal de passage d'air Ca, Cb, Cc respectif lors de la respiration de l'utilisateur. Typiquement, une telle ampoule est configurée pour être alimentée par un courant de 100 mA et soumise à une tension de 14 V.

Le capteur de mesure de flux d'air 4 peut être obtenu à partir d'une ampoule à incandescence ayant une espérance de vie moyenne de 50 000 heures (sous vide), ce qui permet d'assurer une durée de vie satisfaisante de l'appareil de mesure 1. De plus, comme il sera vu par la suite, un capteur défectueux peut être facilement remplacé.

L'épaisseur du filament métallique 41 détermine la consommation en courant du capteur de mesure de flux d'air 4. Typiquement, l'épaisseur du filament métallique 41 est comprise entre 50 μm et 200 μm.

La longueur du filament métallique 41 détermine la tension aux bornes du capteur de mesure de flux d'air 4. Typiquement, la longueur du filament métallique 41 est supérieure à 50 cm, de préférence supérieure à 1 m. Bien entendu, comme cela est représenté sur la figure 6, un tel filament métallique 41, conformément à son agencement initial dans l'ampoule, peut être au moins partiellement enroulé en serpentin ou être au moins partiellement hélicoïdal, et ce, afin de diminuer son encombrement relativement à sa longueur. Un tel filament métallique 41 permet un échange de surface beaucoup plus important, et donc une sensibilité et une amplitude de réponse accrues.

Le filament métallique 41 a entre ses deux extrémités une résistance électrique, qui varie en fonction de la température, et génère une puissance par effet Joule dépendant du flux d'air s'écoulant autour du filament métallique 41, typiquement traversant les spires du filament métallique 41 enroulé.

Le filament métallique 41 peut être en métal noble, par exemple en or, en platine ou dans un alliage de platine et d'iridium. L'utilisation d'un tel métal permet d'augmenter la résistance du capteur de mesure de flux d'air 4 à la corrosion et à l'oxydation et augmente la durée de vie de l'appareil de mesure 1.

De préférence, le filament métallique 41 peut être en tungstène ou en acier. De tels métaux sont couramment utilisés dans les filaments d'ampoules à incandescence et sont peu onéreux. Cela permet d'avoir un appareil 1 plus accessible pour les praticiens. De préférence, le filament métallique 41 est oxydé au préalable, par exemple brûlé sur un support de test avant assemblage sur la carte électronique 6. Cela permet qu'il soit partiellement corrodé et ainsi résistant à l'oxydation.

De préférence, le capteur de mesure de flux d'air 4 est de classe C-2F de sorte que le filament métallique 41 est maintenu en position dans le canal de passage de flux d'air Ca, Cb, Cc respectif par un double support 42. Cela permet d'éviter que le filament métallique 41 ne se déforme ou ne rompe à cause de l'écoulement du flux d'air dans le canal de passage d'air Ca, Cb, Cc où il est agencé.

Le capteur de mesure de flux d'air 4 comprend également un corps de capteur 44 présentant deux contacteurs 43 reliant chacun une extrémité du filament métallique 41 à une électrode (ou patte) 45 respective. Le filament métallique 41 peut être connecté aux contacteurs 43 par soudage ou sertissage.

Le capteur de mesure de flux d'air 4 est destiné à être connecté au circuit électrique de la carte électronique 6 par l'intermédiaire des deux électrodes 45, afin de faire passer dans le filament métallique 41 un courant électrique. Ainsi, chaque capteur de mesure de flux d'air 4a, 4b, 4c est propre à être alimenté par un courant électrique lors d'une respiration de l'utilisateur à travers l'appareil de mesure 1. La structure des capteurs de mesure de flux d'air 4a, 4b, 4c et leur assemblage seront détaillés par la suite.

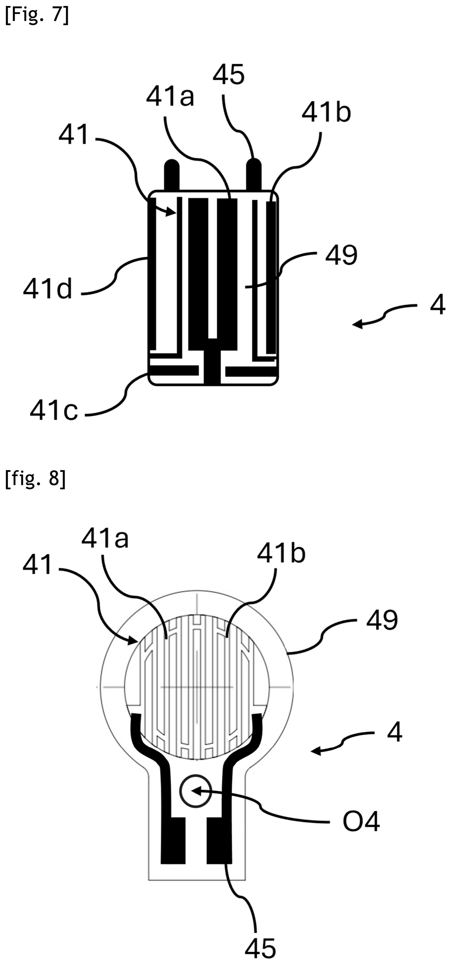

Dans un deuxième mode de réalisation illustré à la figure 7, le capteur de mesure de flux d'air 4 peut être un capteur MAF (sigle de « *mass air flow* » en langue anglaise) selon une technologie éprouvée dans de nombreux domaines (automobile, dispositifs médicaux...). Un tel capteur permet d'obtenir des mesures de vitesse de flux d'air (anémométrie) mais également des mesures de pertes de chaleur (calorimétrie) et de température. Il est configuré pour effectuer une mesure de flux d'air de manière précise et bidirectionnelle.

Le filament métallique 41 peut ne pas être directement au contact du flux d'air mais intégré dans un substrat 49 avantageusement thermiquement conducteur, en d'autres termes à forte conductivité thermique, par exemple un substrat en céramique. Ainsi, le flux d'air ne s'écoule pas tout autour du filament métallique 41, mais autour du capteur de mesure de flux d'air 4 s'étendant dans un plan.

De préférence, le filament métallique 41 comprend une piste en platine, de préférence de pureté élevée.

Le filament métallique 41 est avantageusement formé par une pluralité de pistes résistives 41a, 41b, 41c formant une couche fine sur le substrat 49.

Plus précisément, le filament métallique 41 comprend une première piste résistive 41a, disposée dans une zone centrale du substrat 49, deux pistes résistives latérales 41b, 41d disposées de part et d'autre de la première piste résistive 41a.

La première piste résistive 41a présente une forme de U dont chaque branche a une largeur supérieure aux autres pistes résistives et présente une plus faible résistance électrique. La première piste résistive 41a permet le chauffage et le préchauffage des autres pistes résistives 41b, 41d utilisées pour la volumétrie.

Le filament métallique 41 formant les deux pistes résistives latérales 41b, 41d a une épaisseur variable. En d'autres termes, la deuxième piste résistive 41b a une largeur supérieure à la largeur de la troisième piste résistive 41d. Cela permet de déterminer le sens du flux d'air et la vitesse du flux d'air. Les deux pistes résistives latérales 41b, 41d présentent une résistance électrique supérieure à celle de la première piste résistive 41a.

De préférence, le filament métallique 41 comprend en outre une quatrième piste résistive 41c disposée à l'extrémité du capteur opposée aux électrodes 45 de connexion. Cette piste supplémentaire permet de mesurer la température du flux d'air, et permet d'améliorer la précision de l'estimation du débit.

Le filament métallique 41 peut être fabriqué selon plusieurs étapes, comprenant notamment un revêtement par centrifugation d'une résine photosensible, l'illumination de la résine à travers un masque, le développement de la résine, et une gravure du métal, typiquement du platine, de sorte à ne laisser que la structure des pistes résistives 41a, 41b, 41c, 41d sur le substrat conducteur 49. Chaque piste résistive 41a, 41b, 41c, 41d peut être ajustée individuellement au laser pour obtenir une résistance spécifique selon les besoins.

De préférence, le substrat 49 est recouvert par une fine couche de protection recouvrant les pistes résistives 41a, 41b, 41c, 41d. La fine couche de protection a typiquement une épaisseur comprise entre 1 μm et 50 μm. Un tel capteur est plus robuste que le premier mode de réalisation, et présente une large plage de fonctionnement.

Ce mode de réalisation permet de placer chaque capteur de mesure de flux d'air 4 dans le canal de passage d'air Ca, Cb, Cc de façon optimale, en adaptant facilement les dimensions de la plaque de substrat 49 à la section du canal de passage d'air Ca, Cb, Cc.

Selon un troisième mode de réalisation, le filament métallique 41 comprend plusieurs pistes résistives rectilignes formant une grille d'un circuit imprimé.

Le capteur de mesure de flux d'air 4 dans ce mode de réalisation peut comprendre une plaque de substrat 49 annulaire supportant le filament métallique 41, laquelle est en forme de grille. La plaque de substrat 49 comporte de préférence des orifices de passage d'air. Ainsi, le flux d'air peut s'écouler à travers le substrat 49, et donc à travers le plan du capteur de mesure de flux d'air 4, autour du filament métallique 41 formant la grille.

Dans la variante illustrée sur la figure 8, la plaque de substrat 49 a ici une forme circulaire et comporte une portion latérale annulaire et une portion centrale en forme de grille. Les filaments métalliques 41a, 41b sont formés par revêtement métallique sur la plaque de substrat 49, par exemple par un procédé de déposition de métal, la portion latérale annulaire étant protégée par un masque. Un tel procédé de fabrication est connu et maîtrisé à l'échelle industrielle.

Le métal utilisé est de préférence du platine, comme expliqué précédemment. Cela permet d'obtenir une faible dispersion mécanique, avec une bonne précision de mesure. La consommation électrique du capteur est également réduite, de l'ordre d'un facteur 10, par rapport au premier mode de réalisation. Cela permet d'augmenter l'autonomie de la sonde 2.

La plaque de substrat 49 est avantageusement souple, et est configurée pour se déformer de manière élastique (c'est-à-dire réversible et sans rupture) sous l'effet du passage du flux d'air. Cela permet d'augmenter la robustesse du capteur et sa durée de vie, et de faciliter l'assemblage. La plaque de substrat 49 est par exemple réalisée en polyimide.

Les électrodes 45 de connexion peuvent être réalisées par des pistes métalliques sérigraphiées reliées au filament électrique 41. Les électrodes 45 sont typiquement en cuivre. Cela permet d'augmenter la robustesse du capteur.

La portion latérale annulaire de la plaque de substrat 49 est de préférence recouverte par un revêtement de parylène. Ce polymère est biocompatible donc adapté à l'utilisation du capteur de mesure de flux d'air 4 dans la sonde 2. Il présente par ailleurs de bonnes propriétés de conformité et d'uniformité, d'isolation électrique et de barrière chimique. Cela permet d'augmenter la durée de vie du capteur de mesure de flux d'air 4 en autorisant une construction étanche du capteur, et donc de permettre le lavage de la sonde 2. La grille formant le filament métallique 41 peut également être recouverte par une couche de protection en parylène. Le principe de base du capteur de mesure de flux d'air 4 est celui d'un anémomètre à fil chaud évaluant la vitesse d'un fluide en mesurant la puissance transférée par le fil métallique 41 chauffé pour abaisser sa température. Le transfert de chaleur dépend de la vitesse d'écoulement du flux d'air le traversant ou s'écoulant autour de la thermistance, selon la loi de King :

$$R \times I^2 = A + B\, v^{0.45},$$

avec R la résistance du filament métallique 41,

　I le courant traversant le filament métallique 41,
　A et B des paramètres dépendant notamment de la température du filament métallique 41, et
　v la vitesse d'écoulement du flux d'air autour du filament métallique 41.

La résistance électrique du filament métallique 41 dépend de plusieurs facteurs. D'une part, la résistance électrique du filament métallique 41 dépend des caractéristiques techniques du filament métallique 41, notamment sa composition, sa longueur effective, sa résistance de contact. Elle augmente avec le vieillissement du filament métallique 41, dont le diamètre réduit avec l'usure. L'augmentation est proportionnelle à la longueur du filament métallique 41, et dépend du type de matériau choisi. Elle est particulièrement notable pour des filaments métalliques en acier ou en tungstène.

D'autre part, la résistance électrique du filament métallique 41 augmente avec la température du filament selon une loi logarithmique. La température du filament métallique 41 augmente lorsqu'un courant traverse le filament métallique 41 à cause des pertes thermiques liées à l'effet Joule, et varie en fonction des conditions ambiantes et du flux d'air s'écoulant dans le canal de passage d'air Ca, Cb, Cc dans lequel le capteur de mesure de flux d'air 4 est agencé (par des phénomènes de conduction et convection).

Ces variations de résistance du filament métallique 41 doivent être prises en compte pour assurer la fiabilité de la mesure de la grandeur représentative du flux d'air.

De préférence, le capteur de mesure de flux d'air 4 est pré-calibré de sorte que ses caractéristiques sont déjà disponibles.

De manière classique, la résistance du filament métallique 41 peut être mesurée par un circuit de mesure comprenant un pont de Wheatstone relié aux extrémités du filament métallique 41. Le pont de Wheatstone comprend un galvanomètre permettant de mesurer une variation de résistance du filament métallique 41. Toutefois, ce dispositif nécessite le réglage préalable d'une résistance variable du pont. A cause de la dérive de la résistance du filament métallique 41 due à son usure et aux variations de température, un tel circuit de mesure ne permet pas une mesure fiable dans le temps. De préférence, quel que soit le mode de réalisation, le filament métallique 41 est revêtu par une couche de protection. La couche de protection peut être en matériau électriquement isolant, par exemple en verre, ou électriquement conducteur. La couche de protection a typiquement une épaisseur comprise entre 1 $\mu$m et 50 $\mu$m. Cela permet de protéger le filament métallique des chocs, ou de l'oxydation, tout en permettant la mesure.

*Asservissement du capteur de mesure de flux d'air 4*

La solution proposée est d'implémenter un asservissement des capteurs de mesure de flux d'air 4a, 4b, 4c afin d'obtenir une mesure fiable de la résistance du filament métallique et donc une estimation fiable de la grandeur représentative du flux d'air s'écoulant dans chacun des canaux de passage d'air Ca, Cb, Cc.

Pour ce faire, la carte électronique 6 comprend un microcontrôleur 61, et une pluralité de circuits de mesure, chaque circuit de mesure étant propre à un canal de passage d'air Ca, Cb, Cc associé. Ainsi, chaque capteur de mesure de flux d'air 4 de la sonde 2 est relié au circuit électrique de la carte électronique 6 par son propre circuit de mesure.

Par la suite, on décrira l'un des circuits de mesure de la carte électronique 6 associé au capteur de mesure de flux d'air 4.

De manière générale, le microcontrôleur 61 est configuré pour déterminer ou estimer une grandeur représentative du flux d'air, typiquement la vitesse d'écoulement ou le débit, à partir de données d'entrée comprenant une mesure de tension. La carte électronique 6 peut comprendre une mémoire configurée pour stocker des instructions ou un programme de commande du circuit de mesure mis en œuvre par le microcontrôleur 61, des données mesurées au cours de l'utilisation de l'appareil de mesure 1, et des données préalablement obtenues, par exemple par calibration.

Chaque circuit de mesure comprend en outre un amplificateur 62. L'amplificateur 62 est configuré pour fournir en sortie une tension amplifiée Vo à partir d'une tension de référence Vref et d'une tension aux bornes du filament métallique 41 du capteur de mesure de flux d'air 4, lorsque le circuit de mesure et donc le capteur de mesure de flux d'air est alimenté.

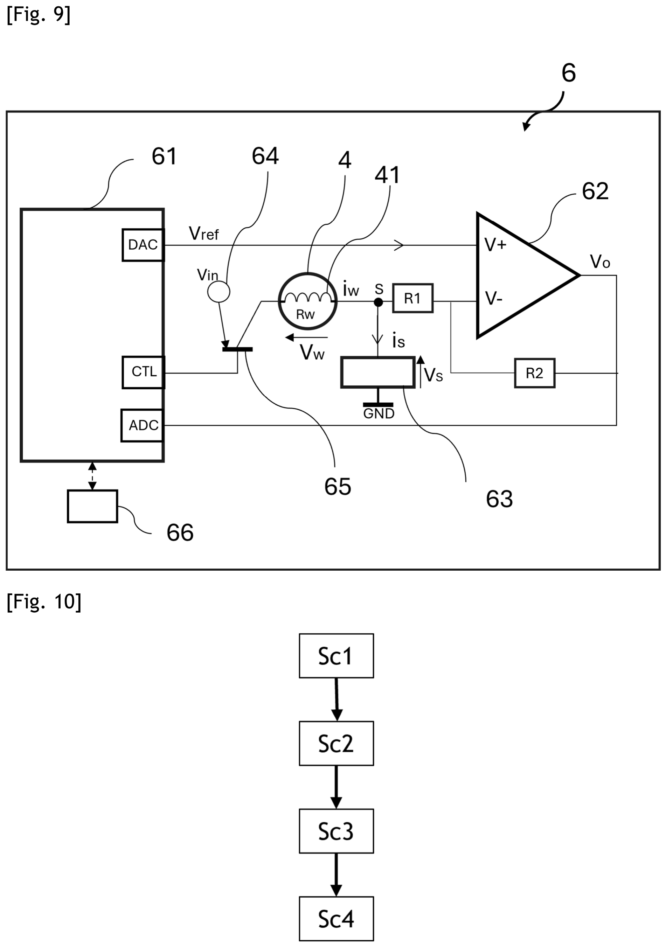

En d'autres termes, l'amplificateur 62 est configuré pour amplifier la tension à une borne du filament métallique 41. Plus précisément, une première entrée au potentiel V⁻ de l'amplificateur 62 est reliée au filament métallique 41. L'amplificateur 62 est sélectionné pour être très stable en température. Typiquement, l'amplificateur 62 est de type sans dérive (« *zero drift* »), avec une dérive de décalage d'entrée de l'ordre du nanovolt par degré Celsius, et un décalage de tension très faible, de préférence inférieur au millivolt. Le microcontrôleur 61 comprend différents modules fonctionnels schématiquement séparés sur la figure 9 en un convertisseur numérique/analogique DAC, un convertisseur analogique/numérique ADC et une unité de commande CTL.

Le convertisseur numérique/analogique DAC est configuré pour fournir la tension de référence Vref à l'amplificateur 62. Il est relié à une deuxième entrée de l'amplificateur 62 au potentiel Vref, typiquement à la borne V+ dans le mode de réalisation illustré.

Le convertisseur analogique/numérique ADC est configuré pour convertir la tension de sortie de l'amplificateur 62 en une valeur numérique. Il est relié à la sortie de l'amplificateur 62.

Dans le mode de réalisation illustré, le circuit de mesure comprend en outre une source de courant 63 (aussi appelée limiteur de courant, puits de courant ou « *current sink* » dans la terminologie anglosaxonne), un générateur de tension 64 et un commutateur 65. Le générateur de tension 64 est relié au filament métallique 41 par l'intermédiaire du commutateur 65.

La source de courant 63 est configurée pour alimenter le circuit de mesure, et pour limiter le courant passant au travers du filament métallique 41. Il est relié à la référence de tension GND (ou masse) et au filament métallique 41. La source de courant 63 est sélectionnée pour ne chauffer que très peu intrinsèquement et donc ne pas conduire à une perte thermique significative.

L'unité de commande CTL est reliée au commutateur 65 et configurée pour actionner le commutateur 65 de sorte à mettre en chauffe le filament métallique 41 et ajuster le courant qui le traverse, par un signal de commande du microcontrôleur 61, avec un rapport cyclique d. Selon la loi des mailles au point S, on a Vw =d x Vᶦⁿ - V$_S$.

Dans le mode de réalisation illustré, l'amplificateur 62 est un amplificateur non inverseur, de sorte que le circuit de mesure comprend une première résistance R1 reliant la borne V- de l'amplificateur 62 au filament métallique 41, et une deuxième résistance R2 reliant la sortie de l'amplificateur 62 à la borne V-. Lorsque l'amplificateur 62 fonctionne en mode linéaire, la tension de sortie Vo vérifie

$$V_o = \frac{1}{\beta}(V_{REF} - (1 - \beta)V_S)$$

avec $\beta = \frac{R_1}{R_1 + R_2}$ le ration des résistances définissant le gain de l'amplificateur 62, et $V_S$ le potentiel au point S, correspondant à la tension de la source de courant 63 (reliée à la masse GND).

Le procédé de commande d'un circuit de mesure de l'appareil de mesure 1, mis en œuvre par le microcontrôleur 61, va maintenant être décrit en référence à la figure 10.

Le filament métallique 41 du capteur de mesure de flux d'air 4 est alimenté en courant I par la source de courant 63. La tension Vw aux bornes du filament métallique 41 varie en fonction de la résistance Rw aux bornes de celui-ci, à courant $i_w$ constant, selon la loi d'Ohm (Vw =Rw x $i_w$).

Au cours d'une **étape Sc1,** le microcontrôleur 61 mesure la tension amplifiée Vo en sortie de l'amplificateur 62 reçue par le convertisseur analogique/numérique ADC.

Au cours d'une **étape Sc2,** le microcontrôleur 61 calcule une amplitude de la mesure de tension amplifiée Vo. Par « amplitude », il est entendu que le microcontrôleur 61 détermine une valeur de tension correspondant à un maximum local sur une plage déterminée. Comme il sera illustré par la suite, l'amplitude calculée peut correspondre à un pic de tension mesuré entre une inspiration et une expiration de l'utilisateur dans le canal de passage d'air où est situé le capteur de mesure de flux d'air 4.

Au cours d'une **étape Sc3,** le microcontrôleur 61 modifie la tension de référence Vref en fonction de la mesure de tension amplifiée Vo, de sorte à maintenir l'amplitude du maximum local constante. En d'autres termes, le microcontrôleur 61 asservit l'amplitude calculée précédemment. Le microcontrôleur 61 est en outre configuré pour faire varier la tension de référence Vref, en fonction de la température, des paramètres de calibration et de la mesure de tension amplifiée Vo. Le microcontrôleur 61 commande, de manière cyclique, la tension de référence Vref du signal d'amplification fournie par le convertisseur numérique/analogique DAC à l'amplificateur 62.

En particulier, le microcontrôleur 61 est configuré pour fixer Vref de sorte que l'inégalité 0 < Vo < Vin soit toujours vérifiée, quelles que soient les conditions, et notamment $(1 - \beta)V_w + V_{REF} < V_{in}$ et

$$(1-\beta)R'_w i_w + V_{REF} < V_{in}$$ , avec $R'_w$ la résistance du filament chauffé sans présence de flux d'air.

Le seuil de détection du circuit de mesure dépendant de la tension de référence Vref, la mesure de résistance reste fiable dans le temps. En effet, il est ainsi possible permet de corriger une dérive de la mesure de tension, observée lorsque la température du filament métallique 41 varie, et donc d'avoir un circuit de mesure et donc un appareil de mesure 1 plus fiable.

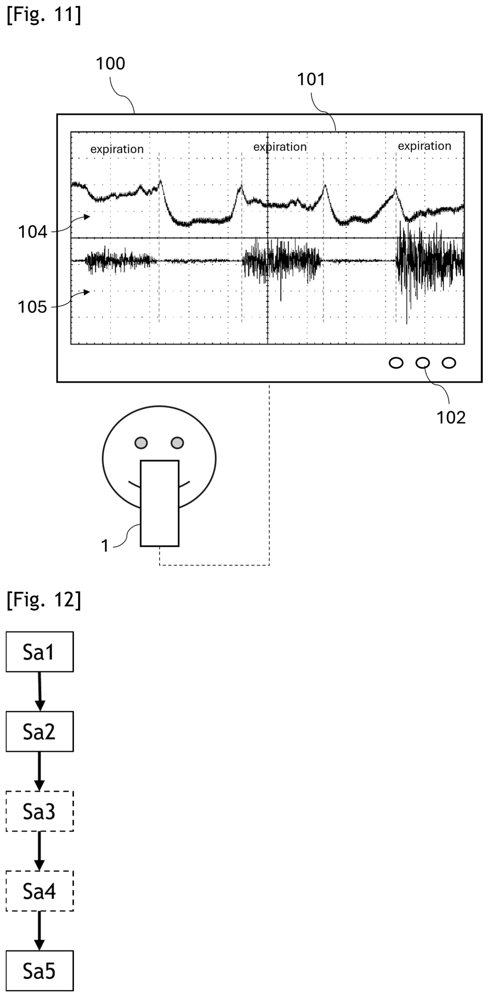

Par exemple, la figure 11 représente des valeurs de tension mesurée par le microcontrôleur 61 au cours de trois cycles de respiration de l'utilisateur d'intensité variable. Avant chaque expiration, on observe un maximum local dans la mesure. Le microcontrôleur 61 est configuré pour réguler la tension de référence Vref de sorte à maintenir une amplitude de la tension amplifiée Vo mesurée en sortie de l'amplificateur 62 constante dans le temps. En d'autres termes, le microcontrôleur 61 pilote le convertisseur numérique/analogique DAC de sorte que le maximum local à une même valeur dans le temps. Ainsi, le circuit de mesure a une sensibilité constante dans le temps.

Au cours d'une **étape Sc4,** le microcontrôleur 61 estime une grandeur représentative du flux d'air s'écoulant dans le canal de passage d'air Ca, Cb, Cc associé au capteur de mesure de flux d'air 4a, 4b, 4c alimenté à partir de la mesure de tension amplifiée Vo. A partir de la mesure de tension amplifiée Vo, et de l'intensité du courant électrique $i_w$ connue par le microcontrôleur 61, le microcontrôleur 61 peut calculer la résistance du filament métallique et la puissance dissipée Rw x $i_w^2$. L'estimation de la grandeur représentative, par exemple la vitesse d'écoulement, peut être réalisée par exemple à partir de tables ou de lois stockées dans la mémoire du microcontrôleur. Typiquement, la grandeur représentative est estimée à partir d'une loi reliant une résistance du filament métallique 41 du capteur actif à une valeur de température, la loi ayant été obtenue par une calibration préalable du circuit de mesure.

Le microcontrôleur 61 peut effectuer des mesures de manière cyclique pendant le fonctionnement de la sonde 2. Par « de manière cyclique », il est compris selon un schéma périodique se reproduisant dans le temps selon une période. La période peut être fixée, par exemple 1 s, ou dépendre du rythme de respiration de l'utilisateur.

De préférence, le microcontrôleur 61 calcule une amplitude de signal. L'amplitude de signal est calculée selon un cycle comprenant une période qui dépend de l'estimée de la grandeur représentative obtenue à l'étape Sc4. Typiquement, la période peut correspondre à la durée d'une expiration ou d'une inspiration, ou à la durée d'un cycle expiration-inspiration. L'estimée de la grandeur représentative, typiquement la vitesse, permet de détecter ces variations respiratoires.

De manière alternative ou complémentaire, la période peut être dépendre des signaux de mesure du microphone 5a, 5b, 5c détectant la variation de pression dans le canal de passage d'air Ca, Cb, Cc associé au capteur de mesure de flux d'air 4 du circuit de mesure considéré. Cela permet d'établir plus précisément la valeur de l'amplitude du signal, en identifiant le maximum local de tension observé entre une inspiration et une expiration dans la voie respiratoire concernée.

Le microcontrôleur 61 peut modifier la tension de référence Vref selon un cycle présentant la même période. En effet, selon que le filament métallique 41 est dans un flux d'air inspiré ou expiré, la température du flux d'air varie, ce qui peut impacter la mesure.

Le fonctionnement du circuit de mesure va maintenant être décrit, en référence à la figure 9.

De préférence, la source de courant 63 délivre un courant électrique de sorte à alimenter le filament métallique 41 du capteur de mesure de flux d'air 4 avec une intensité constante. D'après la loi des nœuds au point S, on a $i_w \approx i_S$ car peu de courant pénètre dans l'amplificateur 62. La source de courant 63 fonctionne comme un puits de courant.

Le microcontrôleur 61, ici via l'unité de commande CTL, peut actionner le commutateur 65 entre une position ouverte et une position fermée. Lorsqu'il est en position fermée, le potentiel imposé par le générateur de tension 64 à une extrémité du filament métallique 41 est $V^{in}$. Ainsi, le filament métallique 41 est traversé par des impulsions de courant (pulse) dont la fréquence varie selon le signal de commande, et notamment selon le rapport cyclique d du commutateur 65.

L'intensité moyenne du courant traversant le filament métallique 41 est donc pilotée par le microcontrôleur 61. De préférence, le microcontrôleur 61 est en outre configuré pour modifier l'intensité du courant électrique délivré à partir de la mesure de tension amplifiée Vo. Cela permet d'augmenter le courant électrique $i_w$ lorsque la résistance du filament métallique 41 augmente, à cause de l'usure ou de la température, et donc de garantir que la tension aux bornes du filament métallique 41, en entrée de l'amplificateur 62, est suffisamment élevée. Cette modification peut avoir lieu notamment au cours du préchauffage décrit par la suite.

Comme évoqué précédemment, le microcontrôleur 61 est configuré pour prendre en compte la variation de résistance électrique du filament métallique 41 dans l'estimation de la grandeur représentative au cours de l'étape Sc4.

De préférence, le microcontrôleur 61 intègre une compensation des variations de résistance due à la température via une calibration préalable du circuit de mesure. En d'autres termes, une calibration du circuit de mesure est préalablement mise en œuvre par un opérateur avant assemblage de la carte électronique 6 sur l'appareil de mesure 1. Par exemple, des mesures de résistance du filament métallique 41 et de la tension amplifiée Vo sont réalisées pour des débits de flux d'air connu et des températures ambiantes connues, et pour une intensité de courant $i_w$ dans le filament métallique 41 connue. Cela permet de prendre en compte l'impact de l'évolution de la température du filament métallique 41 sur la résistance au cours de l'estimation de la vitesse d'écoulement du flux d'air à partir de la mesure de tension amplifiée. Notamment, la résistance $R''_w$ aux bornes du filament métallique 41 en présence de flux d'air est inférieure à la résistance $R'_w$ en l'absence de flux d'air. De même, on observe lors de chaque expiration que la mesure de tension amplifiée atteint un plateau dont la valeur moyenne est de plus en plus faible. De préférence, le microcontrôleur 61 est configuré pour asservir la tension de référence Vref et/ou du rapport cyclique d du commutateur 65 de sorte à maintenir la valeur moyenne de tension au cours d'une expiration stationnaire ou constante dans le temps. Cela évite également une dérive de la mesure et permet une meilleure fiabilité de l'appareil de mesure 1.

De préférence, le microcontrôleur 61, via un signal de veille émis par l'unité de commande CTL, ouvre le commutateur 65 lorsque l'appareil de mesure 1 n'est pas utilisé. Le microcontrôleur 61 peut alors ouvrir le commutateur 65 de chaque circuit de mesure simultanément. Cela permet avantageusement de ne pas faire passer de courant dans le filament métallique 41 et donc de ne pas participer à son usure de manière non nécessaire. Cela permet de mettre l'appareil de mesure 1 en veille rapidement et également de ne pas consommer la puissance disponible dans la batterie éventuelle, et donc de préserver l'autonomie de l'appareil de mesure 1.

De préférence, le microcontrôleur 61 peut piloter indépendamment les commutateurs respectifs de chaque circuit de mesure. Par exemple, lorsque l'appareil de mesure 1 est utilisé pour mesurer uniquement la respiration buccale de l'utilisateur, le microcontrôleur 61 peut ouvrir les commutateurs des circuits de mesure associés au premier capteur de mesure de flux d'air 4a, et au deuxième capteur de mesure de flux d'air 4b. Ainsi, cela permet de ne pas consommer inutilement les filaments métalliques 41 des capteurs de mesure de flux d'air 4a, 4b.

De préférence, le microcontrôleur 61 est en outre configuré pour commander un préchauffage du filament métallique 41 d'au moins un capteur de mesure de flux d'air 4a, 4b, 4c pendant une durée prédéterminée avant de mesurer la tension amplifiée Vo. Comme précédemment, le microcontrôleur 61 peut commander le préchauffage au niveau de tous les circuits de mesure, ou de manière sélective, selon l'utilisation de l'appareil de mesure 1.

Le circuit de mesure ne comprenant pas d'autres pertes significatives que celles induites par le filament métallique 41, malgré la présence des résistances R1,R2 et de résistances internes à la source de courant 63. Le circuit de mesure présente une bonne réactivité, c'est-à-dire une bonne réponse dynamique. Cela est due à la nature du filament métallique, notamment à la longueur et à l'enroulement. La durée prédéterminée pour chauffer le filament métallique 41 peut être courte, par exemple égale à 5 s toutes les minutes, ou encore 1 s toutes les 5 secondes, ou selon un autre exemple 5 s toutes les 25 s. La durée de préchauffage peut être stockée en mémoire. Elle peut être déterminée au cours de la calibration préalable.

Typiquement, le microcontrôleur 61 effectue périodiquement un préchauffage du filament métallique 41 avant de démarrer les mesures de la tension amplifiée. Cela permet de stabiliser la résistance du filament et de limiter les variations au cours de la mesure, et donc de réduire l'incertitude d'estimation de la vitesse ou du débit du flux d'air.

Par exemple, le microcontrôleur 61 peut recevoir un signal d'activation indiquant que l'appareil de mesure 1 va être utilisé, et commander le commutateur 65 en position fermée à la réception de ce signal d'activation. Le signal d'activation peut provenir d'un système externe, par exemple d'une interface ou d'une application à laquelle peut se connecter l'appareil de mesure 1 en Bluetooth (marque déposée). Le signal d'activation peut provenir directement de l'utilisateur, par exemple via un bouton agencé sur la coque 21 ou via des capteurs de mouvements intégrés à l'appareil de mesure 1. Typiquement, le signal d'activation peut être émis 10 s avant le démarrage d'un jeu.

De manière alternative, le microcontrôleur 61 peut comprendre une horloge interne configurée pour émettre le signal de préchauffage du filament métallique 41 selon un cycle prédéfini.

De préférence, au cours de la phase de préchauffage, le microcontrôleur 61 ajuste le courant I traversant le filament métallique 41 pour étalonner précisément la résistance du filament métallique 41. En d'autres termes, le microcontrôleur 61 est configuré pour modifier l'intensité du courant délivré par la source de courant 63 et/ou pour modifier la fréquence d'actionnement du commutateur 65 de sorte que la tension amplifiée Vo atteigne une valeur cible lors de la phase de préchauffage. Cela permet d'augmenter la précision de la mesure de tension, et donc l'estimation de la grandeur caractéristique.

De préférence, le microcontrôleur 61 comprend en outre une horloge interne configurée pour mesurer une durée d'alimentation du filament métallique 41 du capteur de mesure de flux d'air 4 alimenté. Le microcontrôleur 61 peut stocker un compteur associé à chaque capteur de mesure de flux d'air 4a, 4b, 4c, s'incrémentant quand le circuit de mesure correspondant est alimenté.

Le microcontrôleur 61 peut alors émettre un signal de maintenance lorsque la durée d'alimentation mesurée dépasse une durée maximale d'utilisation. Ainsi, le microcontrôleur 61 peut transmettre à l'utilisateur une information pour planifier un remplacement du capteur de mesure de flux d'air 4 lors d'une opération de maintenance à partir de la durée d'utilisation mesurée. Typiquement, le microcontrôleur 61 peut transmettre, via l'interface de l'unité de traitement 100 à laquelle l'appareil de mesure 1 est connecté, un message d'alerte lorsque le temps d'utilisation du capteur de mesure de flux d'air 4 dépasse un temps limite, par exemple 1000 h. Cela permet de ne pas utiliser l'appareil de mesure 1 lorsque la fiabilité de la mesure est compromise, et donc de ne pas produire de diagnostic orthophonique inconsistant par exemple.

De préférence, la tension générée par le générateur de tension 64 est faible, par exemple inférieure à 5 V, typiquement comprise entre 3 V et 4 V, par exemple égale à 3,3 V. L'intensité du courant générée ou limitée par la source de courant 63 est faible, par exemple inférieure à 150 mA. De préférence, l'intensité du courant est inférieure à 100 mA, typiquement égale à 50 mA (en moyenne). Cela permet que la puissance dissipée par le filament métallique 41 et consommée par le circuit de mesure soit réduite. L'adaptation du courant délivré à la tension mesurée Vo permet de préserver la sensibilité du circuit de mesure, et de réduire la consommation de l'appareil de mesure 1, ce qui augmente son autonomie.

De préférence, la sonde 2 comprend en outre un capteur de température 66 configuré pour fournir une mesure de température au microcontrôleur 61. De préférence, chaque circuit de mesure de la carte électronique 6 comprend un capteur de température 66 relié au microcontrôleur 61. Cela permet au microcontrôleur 61 de déterminer mesurer la variation de température du filament métallique 41, et d'estimer la valeur représentative du flux d'air s'écoulant dans le canal de passage d'air Ca, Cb, Cc associé en prenant compte de cette information.

Par exemple, le capteur de température 66 est un capteur à puce retournée en platine pouvant être assemblée sur la carte électronique 6 par soudage ou collage. Un tel capteur peut opérer sur une large plage de température, typiquement entre -50°C et 150°C. Il a l'avantage d'être très compact, et présente un temps de réponse très rapide et une grande stabilité dans le temps. Selon un autre exemple, le capteur de température 66 est une thermistance linéaire à couche mince de nickel. Un tel capteur de température 66 présente une plage opérative similaire et peu de variabilité avec l'évolution de la température, ce qui permet une mesure stable. Selon un autre exemple, le capteur de température 66 est un capteur en platine à fil pouvant être soudé sur la carte électronique 6. Un tel capteur de température 66 présente une plus grande plage opérative, typiquement jusqu'à 600°C, et également une grande stabilité dans le temps (erreur inférieure à 0.04 % après 1000 h à la température d'opération maximale).

Dans ce mode de réalisation, le microcontrôleur 61 est en outre configuré pour mesurer la température de manière cyclique via le capteur de température 66. Cela permet de prendre en compte l'impact de la température ambiante ou de la température du flux d'air, qui varie entre l'inspiration et l'expiration, sur l'estimation de la grandeur représentative.

De préférence, les capteurs de mesure de flux d'air 4 sont configurés pour réaliser un auto-étalonnage, de sorte que l'appareil de mesure 1 permet d'effectuer des mesures de respiration de l'utilisateur sans étalonnage préalable.

Le circuit de mesure présenté schématiquement comprend un seul filament métallique 41. La carte électronique 6 étant reliée aux trois capteurs de mesure de flux d'air 4a, 4b, 4c, on comprendra que le circuit de mesure représenté est dupliqué de sorte que chacun des filaments métalliques 41 respectifs des capteurs de mesure de flux d'air 4a, 4b, 4c est intégré dans un circuit de mesure.

De préférence, le microcontrôleur 61 pilote chacun des capteurs de mesure de flux d'air 4a, 4b, 4c de manière indépendante. Chaque capteur de mesure de flux d'air 4a, 4b, 4c peut être intégré dans un circuit de mesure indépendant, de sorte que le microcontrôleur 61 reçoit une mesure de tension amplifiée pour chacun des capteurs de mesure de flux d'air 4a, 4b, 4c de la sonde 2. Le microcontrôleur 61 est configuré pour calculer ou estimer la grandeur représentative d'un flux d'air nasal ou buccal à partir du signal de mesure, selon la provenance du signal de mesure.

De préférence, les capteurs de mesure de flux d'air 4a, 4b, 4c présentent une forte sensibilité, de sorte que le microcontrôleur 61 peut estimer un débit de flux d'air sur une plage d'amplitude supérieure à ± 15 l/s, avec une précision inférieure à ± 5 % d'erreur, de préférence inférieure à ± 1 % d'erreur. Avantageusement, la plage de débit mesurable par les capteurs de mesure de flux d'air est de ± 250 slm (litre standard par minute). La résolution des capteurs de mesure de flux d'air 4a, 4b, 4c est de préférence inférieure à 0,1 slm, de préférence encore inférieure à 0,01 slm.

Les signaux de mesure des capteurs de mesure de flux d'air 4a, 4b, 4c peuvent permettre au microcontrôleur 61 de calculer une pluralité de grandeurs représentatives, afin d'aider le praticien dans son diagnostic. Par exemple, le microcontrôleur 61 peut être configuré pour calculer à partir des signaux de mesure, une valeur de ventilation maximale par minute (MW), un débit expiratoire de pointe (PEF), un volume d'air expiré ou inspiré.

Au moins un parmi les capteurs de mesure de flux d'air 4a, 4b, 4c peut être configuré pour mettre en œuvre la technologie de spirométrie TrueFlow (marque déposée). Un autre exemple de capteur de mesure de flux d'air 4a, 4b, 4c est commercialisé par Sensirion (marque déposée) et basé sur la technologie CMOSens®.

*Assemblage industriel des capteurs de mesure de flux d'air* 4a, 4b, 4c Afin d'utiliser les ampoules à incandescence décrites précédemment comme capteurs de mesure de flux d'air 4a, 4b, 4c dans le circuit de mesure, un procédé d'assemblage est proposé. En effet, le filament métallique 41 du capteur de mesure de flux d'air 4a, 4b, 4c est très fragile.

De préférence, le capteur de mesure de flux d'air 4 est monté sur un circuit imprimé modulaire 46 rapporté sur la carte électronique 6. Cela permet un assemblage sans risque des capteurs de mesure de flux d'air 4a, 4b, 4c dans l'appareil de mesure 1, mais aussi un remplacement simple des capteurs de mesure de flux d'air 4a, 4b, 4c en cas de défaillance, ou lorsqu'une durée d'utilisation maximale des capteurs est dépassée. La fiabilité de l'appareil de mesure 1 est ainsi améliorée.

De préférence encore, tous les capteurs de mesure de flux d'air 4a, 4b, 4c sont assemblés sur le circuit imprimé 46 modulaire connecté de manière amovible à la carte électronique 6.

Le procédé d'assemblage de l'appareil de mesure 1 dans le mode de réalisation à trois capteurs est illustré sur les figures 12 à 17.

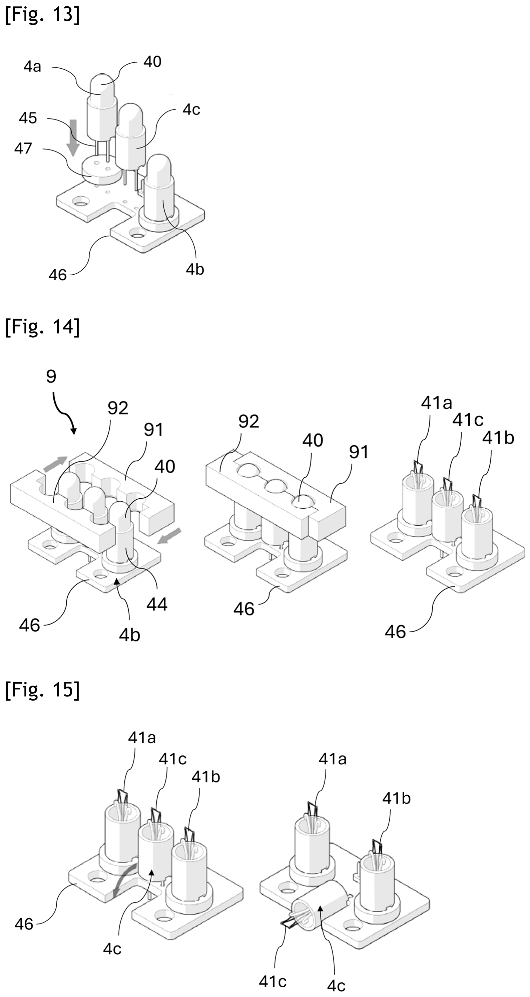

En référence à la figure 13, au cours d'une **étape d'assemblage Sa1,** une pluralité d'ampoules à incandescence sont assemblées sur le circuit imprimé 46. Chaque ampoule à incandescence comprend le bulbe de protection 40 recouvrant le filament métallique 41 qui est destiné à devenir l'élément sensible du capteur de mesure de flux 4a, 4b, 4c. En d'autres termes, les ampoules à incandescence sont montées sur le circuit imprimé modulaire 46. Le bulbe de protection 40 en verre est conçu pour protéger le filament métallique 41 et éventuellement contenir un gaz adapté.

Le circuit imprimé 46 comprend pour chaque capteur de mesure de flux d'air 4a, 4b, 4c deux orifices traversant configurés pour le passage des électrodes 45 du capteur de mesure de flux d'air 4a, 4b, 4c. Une soudure ou un collage peut être réalisé pour assurer la connexion électrique entre les électrodes 45 et le circuit imprimé 46.

De préférence, le capteur de mesure de flux d'air 4a, 4b peut être assemblé sur le circuit imprimé 46 en utilisant un joint d'étanchéité 47. Un tel joint d'étanchéité 47 permet d'éviter les problèmes de couplage et un échappement d'air en dehors du canal de passage d'air Ca, Cb, Cc où est agencé le capteur de mesure de flux d'air. Le joint d'étanchéité 47 permet également de surélever le corps de capteur 44 et d'assurer que le filament métallique 41 du capteur de mesure de flux d'air 4a, 4b sera bien positionné dans le canal de passage d'air Ca, Cb correspondant.

En référence à la figure 14, au cours d'une **étape de retrait Sa2,** le bulbe de protection 40 de chaque ampoule à incandescence est retiré de sorte à obtenir la pluralité de capteurs de mesures de flux d'air 4a, 4b, 4c.

De préférence, tous les bulbes de protection sont découpés simultanément par un dispositif 9. Le dispositif 9 comprend deux membres 91, 92 présentant une forme complémentaire aux capteurs de mesure de flux d'air 4a, 4b, 4c assemblés, de sorte à retirer, après découpage, chaque bulbe de protection 40 sans risque d'éclat. Après découpage, les filaments métalliques 41a, 41b, 41c des capteurs de mesures de flux d'air 4a, 4b, 4c sont libres et peuvent être traversés par un flux d'air pour permettre l'estimation de la grandeur représentative.

En référence à la figure 15, au cours d'une **étape facultative de pliage Sa3,** un capteur de mesure de flux d'air 4c est plié. Dans le mode de réalisation de l'appareil de mesure 1 illustré, le capteur de mesure de flux d'air 4c buccal est plié. En effet, le troisième canal de passage d'air Cc s'étend selon la direction de flux buccal X, sensiblement orthogonale à la direction de flux nasal Z. Ainsi, le pliage permet que le capteur de mesure de flux d'air 4c buccal soit orienté pour que son filament métallique 41c soit traversé par le flux d'air buccal après assemblage du circuit imprimé 46 sur la carte électronique 6 de la sonde 2.

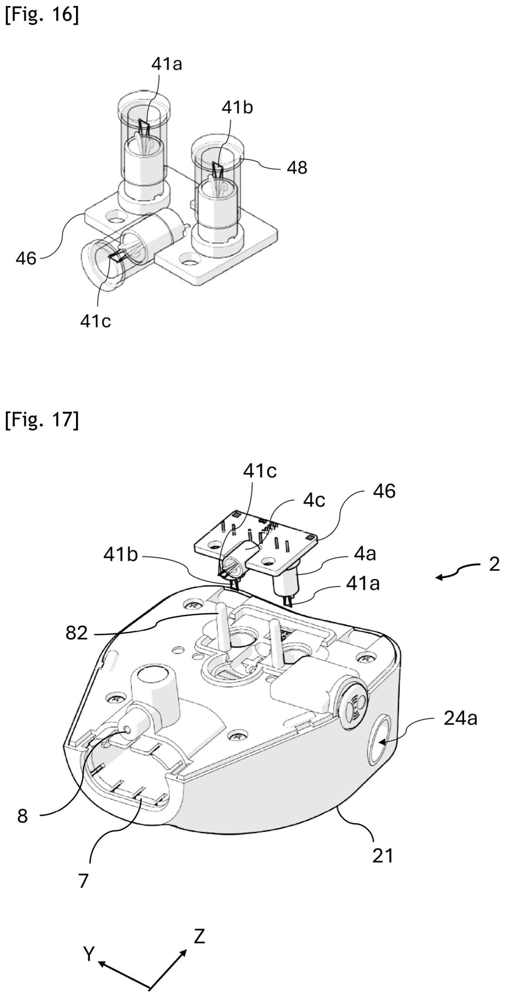

En référence à la figure 16, le procédé d'assemblage comprend une **étape de protection Sa4 facultative,** au cours de laquelle un couvercle amovible 48 est disposé sur chaque capteur de mesure de flux d'air 4a, 4b, 4c, de sorte à protéger leur filament métallique 41. Cela permet que le circuit imprimé 46 obtenu après pliage, donc prêt à être assemblé sur la carte électronique 6 de la sonde 2, soit stocké ou déplacé sans risque d'abîmer le filament métallique 41, et donc de préserver l'intégrité du capteur de mesure de flux d'air 4a, 4b, 4c.

En référence à la figure 17, le circuit imprimé 46 portant la pluralité de capteurs de mesures de flux d'air 4a, 4b, 4c peut être intégré sur la carte électronique 6 de la sonde 2 au cours d'une **étape d'intégration Sa5.**

Dans le mode de réalisation illustré, le circuit imprimé 46 comprend des orifices de fixation destinés à recevoir des plots de fixation 82 de la coque 21, de sorte à maintenir le circuit imprimé fixement après intégration. La coque 21 délimite des orifices circulaires débouchant sur les canaux de passage d'air Ca, Cb nasaux au niveau desquels sont insérés les capteurs de mesure de flux d'air 4a, 4b nasaux. La coque 21 délimite un orifice débouchant sur le troisième canal de passage d'air Cc au niveau duquel est inséré le capteur de mesure de flux d'air 40 buccal.

Le circuit imprimé 46 comprend une face inférieure, opposée à la face sur laquelle sont assemblés les capteurs de mesure de flux d'air 4. Les pattes 45 des capteurs de mesure de flux d'air 4a, 4b débouchent sur la face inférieure. La face inférieure présente des connecteurs pour assurer la connexion électrique du circuit imprimé 46 sur la carte électronique 6.

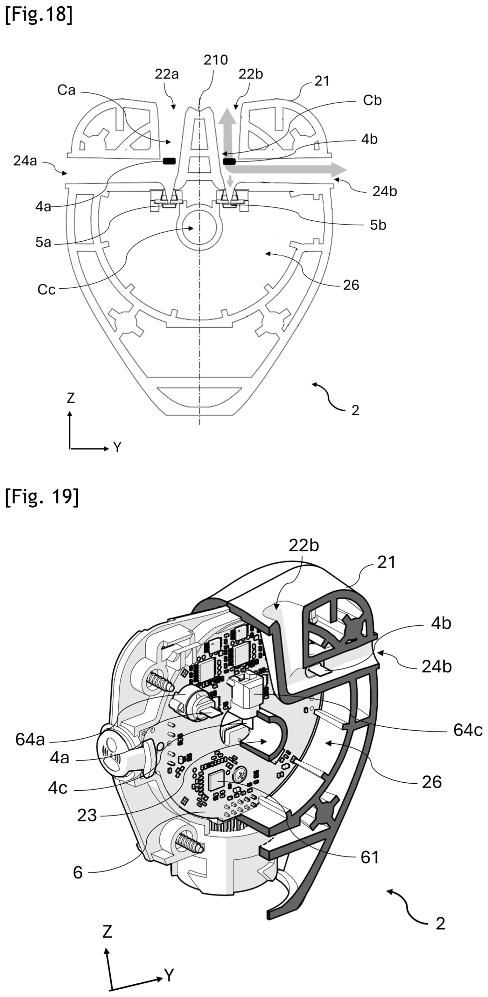

En référence aux figures 17 et 18, on comprendra que le plan des capteurs dans le premier mode de réalisation est normal à la direction du flux d'air dans le canal de passage d'air respective. Le filament métallique 41 peut s'étendre dans la première portion ou dans la deuxième portion.

On comprendra que, bien que le procédé d'assemblage soit illustré dans le premier mode de réalisation des capteurs de mesure de flux d'air 4, un procédé d'assemblage similaire peut être implémenté pour des capteurs tels qu'illustrés aux figures 7 et 8, comprenant une étape d'assemblage des substrats conducteurs 49 sur le circuit imprimé 46 et une étape facultative de pliage Sa3. On comprendra également que l'étape de retrait Sa2 n'est pas nécessaire.

De manière alternative, les capteurs de mesure de flux d'air 4a, 4b, 4c peuvent être disposés sur un support en matière plastique ou thermoformé de sorte que chaque capteur de mesure de flux d'air 4a, 4b, 4c soit directement positionné dans le canal de passage d'air Ca, Cb, Cc respectif, sans nécessiter de pliage.

En référence à la figure 19, le support 64a du capteur de mesure de flux d'air nasal 4a présente une géométrie différente du support 64c du capteur de mesure de flux d'air buccal 4c. Ainsi, le plan du substrat conducteur 49 des capteurs de mesure de flux d'air nasal 4a, 4b s'étend selon dans la portion secondaire dans un plan normal à la direction Z, c'est-à-dire selon la direction du flux d'air, de sorte qu'un flux d'air s'écoulant depuis la première ouverture 22a selon la direction Y traverse successivement les deux pistes résistives 41b, 41d latérales, dans un ordre opposé par rapport à l'inspiration (et de même par la deuxième ouverture 22b).

De manière similaire, le support 64c est agencé dans le canal Cc de sorte que lors de l'inspiration ou expiration buccale, les pistes résistives 41b, 41d latérales du capteur de mesure de flux d'air buccal 4c soient traversées successivement.

Au contraire, le capteur de mesure de flux d'air 4 selon le troisième mode de réalisation est agencé dans le canal de passage d'air respectif de sorte à faire face au flux d'air, le plan de la plaque de substrat 49 étant normal à la direction du flux d'air.

Le capteur dans ce troisième mode de réalisation est de préférence préalablement inséré entre un premier support annulaire et un deuxième support annulaire (non représentés). Cela permet de rigidifier le capteur de mesure de flux d'air et d'éviter une déformation de la grille lors du passage du flux d'air. Le premier support annulaire et le deuxième support annulaire ont une section supérieure à celle de la portion latérale annulaire de la plaque de substrat 49, et un diamètre interne semblable, de sorte à ne pas obstruer le passage du flux d'air sur le filament métallique 41 formé par la grille. Les supports annulaires peuvent comprendre un orifice à aligner avec l'orifice traversant O4 de la plaque de substrat 49, afin de garantir un positionnement dans une orientation fixe du capteur de mesure de flux d'air 4.

Les électrodes 45 s'étendent en dehors du premier et du deuxième support annulaire, afin de permettre la connexion à la carte électronique 6. *Procédé de maintenance des capteurs de mesure de flux d'air 4*

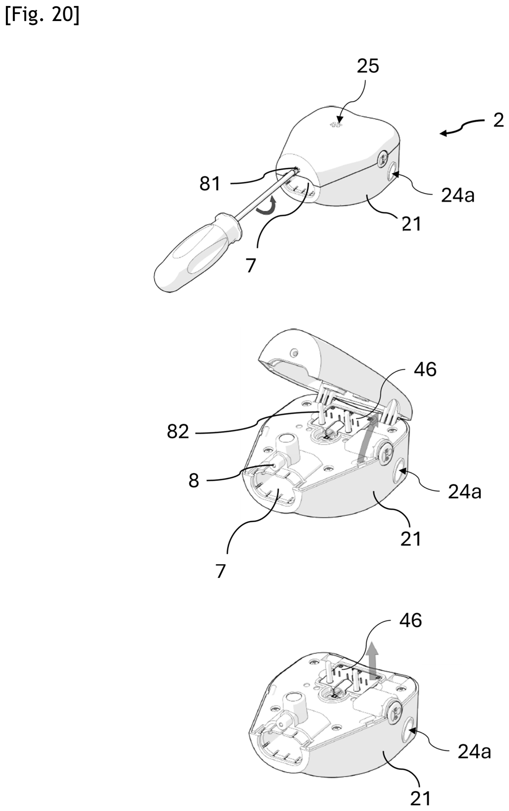

En référence à la figure 20 est illustré un procédé de maintenance de la sonde 2. La coque 21 est ici formée d'une paroi avant présentant la troisième ouverture 23 et les ouvertures latérales 24a, 24b, et d'une paroi arrière présentant l'ouverture arrière 25. Les deux parois de la coque 21 sont amovibles et sont fixées entre elle par une vis 81 inséré dans un orifice de fixation 8 de la coque 21. Après désassemblage de la vis 81, la paroi arrière peut être basculée autour d'un axe de rotation s'étendant selon la direction Y, la paroi avant et la paroi arrière étant reliées entre elles par des charnières.

Le circuit imprimé 46 modulaire défectueux peut alors être retiré de la coque 21 par coulissement le long des plots de fixation 82 de la coque 21. Un procédé inverse peut être suivi pour intégrer un nouveau circuit imprimé 46 à la sonde 2.

La présente invention n'est pas limitée par les moyens de fixation et d'assemblage décrits, des alternatives, telle que clipsage ou vissage pouvant être envisagées.

La poignée 3 peut comprendre un voyant lumineux dont l'allumage est commandé par le microcontrôleur 61. Cela permet de communiquer avec l'utilisateur de l'appareil de mesure 1, par exemple d'indiquer que le niveau de la batterie est faible, ou que la durée d'utilisation maximale des capteurs de mesure de flux d'air 4 est atteinte, de sorte que l'utilisateur est incité à recharger l'appareil de mesure 1 ou à effectuer une opération de maintenance selon le procédé détaillé plus haut.

*Assemblage de l'appareil de mesure 1*

La sonde 2 amovible peut être assemblée sur une extrémité de la poignée 3, par exemple grâce à des moyens de fixation coopérant entre eux, typiquement un système de vissage ou de clipsage. Les moyens de fixation sont réversibles afin de permettre d'enlever la sonde 2 amovible de la poignée 3.

Dans un mode de réalisation, l'appareil de mesure 1 est alimenté électriquement par l'intermédiaire de l'unité de traitement 100 située à l'extérieur de l'appareil de mesure 1 par l'intermédiaire d'un câble d'alimentation. De préférence, la poignée 3 comprend une batterie permettant d'alimenter les capteurs de mesure de flux d'air 4a, 4b, 4c, les microphones 5a, 5b, 5c et la carte électronique 6. Typiquement, la batterie peut être une batterie Lithium-ion. De préférence, la batterie est rechargeable et peut effectuer des cycles de recharge rapide. La batterie peut avoir une capacité supérieure à 1000 mAh, afin de permettre une autonomie suffisante de l'appareil de mesure 1.

Ainsi, la sonde 2 comprend un premier connecteur 7 et la poignée 3 comprend un deuxième connecteur configuré pour être connecté électriquement au premier connecteur 7, lorsque la sonde 2 est assemblée sur la poignée 3. Le premier connecteur 7 est par exemple de type mâle, et le deuxième connecteur de type femelle.

Dans un mode de réalisation, l'interface entre la poignée 3 et la sonde 2 est numérique (et non analogique). Cela permet une connexion presque illimitée de capteurs de différentes sortes de la sonde 2. L'interface numérique offre aussi l'avantage d'être immunisée des bruits ambiants (notamment électromagnétiques). Le connecteur de l'interface numérique est par exemple un composant logiciel enfichable de type « snap-in » permettant de faire un échange à chaud (« hot-swap »). Une telle interface est protégée contre les phénomènes électrostatiques (transitoires) et ne présente aucune tension aux bornes des connecteurs quand la sonde 2 et la poignée 3 sont séparées.

La coopération des connecteurs entre eux peut contribuer au blocage de la sonde 2 sur la poignée 3 dans la position d'utilisation de l'appareil de mesure 1.

*Utilisation de l'appareil de mesure 1 pour la mesure de respirations*

La carte électronique de l'appareil de mesure 1 peut être connectée à l'unité de traitement 100 du signal de mesure. Par exemple, la poignée 3 comprend, en une extrémité inférieure une interface pour la connexion à l'unité de traitement 100. L'interface est par exemple un port de type USB ou USB-C, de sorte à connecter un câble USB ou USB-C relié à l'unité de traitement 100.

De préférence, l'appareil de mesure 1 comprend des moyens de communication sans fil pour communiquer avec l'unité de traitement 100. Par exemple, la carte électronique 6 comprend un module Bluetooth configuré pour échanger des informations en Bluetooth (marque déposée) avec l'unité de traitement 100. De manière alternative ou complémentaire, l'appareil de mesure 1 comprend un module Wi-Fi (marque déposée) configuré pour échanger des informations via Wi-Fi (marque déposée) avec l'unité de traitement 100.

L'unité de traitement 100 comprend avantageusement une interface homme-machine, par exemple un écran 101. L'écran 101 peut être tactile. L'unité de traitement 100 peut comprendre des boutons ou curseurs 102 permettant de sélectionner les signaux à afficher ou de modifier l'affichage sur l'écran 101. Cela permet de visualiser l'évolution temporelle des signaux de mesure en provenance des capteurs de mesure de flux d'air 4a, 4b, 4c et des microphones 5a, 5b, 5c, comme illustré sur la figure 11.

L'appareil de mesure 1 suivant l'invention permet ainsi au praticien d'effectuer une étude simultanée et comparative des flux aériens nasaux et/ou oraux. Notamment, il permet d'établir un bilan clinique de la ventilation aérienne supérieure et des capacités fonctionnelles du voile du palais. L'analyse des signaux de mesure 104 des capteurs de mesure de flux d'air 4a, 4b, 4c, et des signaux de mesure 105 issus des microphones 5a, 5b, 5c permet au praticien d'évaluer les insuffisances vélaires et d'apprécier des déperditions nasales, et de proposer des méthodes de rééducation de la respiration et de la phonation adaptées à l'utilisateur, tel qu'un patient.

L'observation par le praticien (par exemple orthodontiste ou orthophoniste) des courbes temporelles des signaux de mesure sur l'unité de traitement 100 permet d'effectuer un diagnostic sur la respiration de l'utilisateur. L'appareil de mesure 1 permet également d'analyser la respiration buccale et/ou nasale de l'utilisateur lorsqu'il prononce une parole. La détection par l'appareil de mesure 1 d'un flux d'air nasal lors de la répétition de phonèmes, de mots ou de phrases prononcés par l'utilisateur, objective le phénomène de la déperdition nasale et en quantifie la sévérité.

Grâce à la sensibilité des capteurs de mesure de flux d'air 4a, 4b nasal, les mouvements d'ouverture et de fermeture du sphincter vélo-pharyngé sont clairement visibles sur les courbes affichées sur l'unité de traitement 100, objectivant le potentiel musculaire du sphincter et permettant de vérifier sa rigidité, ses possibilités d'ouverture et de fermeture complète, sa rapidité d'exécution des mouvements d'ouverture et de fermeture, ou encore sa stabilité.

La présente invention ne se limite pas aux modes de réalisation présentés. Les modes de réalisation et exemples décrits peuvent être combinés les avec les autres selon toute combinaison techniquement envisageable ou être sélectionnés indépendamment les uns des autres.

## Revendications

**1.** Appareil de mesure (1) de respirations buccales et nasales d'un utilisateur, comprenant une sonde (2) délimitant intérieurement

un premier canal de passage d'air (Ca) comprenant une première ouverture (22a), la première ouverture (22a) étant propre à être positionnée sous une première narine de l'utilisateur, un deuxième canal de passage d'air (Cb) comprenant une deuxième ouverture (22b), la deuxième ouverture (22b) étant propre à être positionnée sous une deuxième narine de l'utilisateur, et un troisième canal de passage d'air (Cc) comprenant une troisième ouverture (23), la troisième ouverture (23) étant propre à être positionnée en vis-à-vis de la bouche de l'utilisateur,

la sonde (2) comprenant

une carte électronique (6) comprenant un microcontrôleur (61) et une pluralité de circuits de mesure, chaque circuit de mesure étant propre à un canal de passage d'air (Ca, Cb, Cc) associé, l'appareil de mesure (1) étant **caractérisé en ce que** chaque circuit de mesure comprend

un capteur de mesure de flux d'air (4a, 4b, 4c) agencé dans le canal de passage d'air (Ca, Cb, Cc) associé et comprenant un filament métallique (41a), autour duquel est propre à s'écouler un flux d'air s'écoulant dans le canal passage d'air (Ca, Cb, Cc) associé, chaque capteur de mesure de flux d'air (4a, 4b, 4c) étant propre à être alimenté par un courant électrique lors d'une respiration de l'utilisateur à travers l'appareil (1), et un amplificateur (62) configuré pour fournir en sortie une tension amplifiée (Vo) à partir d'une tension de référence (Vref) et d'une tension aux bornes du filament métallique (41) du capteur de mesure de flux d'air (4a, 4b, 4c) alimenté,

le microcontrôleur (61) étant en outre configuré pour :

- mesurer la tension amplifiée (Vo) en sortie de l'amplificateur (62),
- calculer une amplitude de signal à partir de la mesure de tension amplifiée (Vo),
- modifier la tension de référence (Vref) en fonction de la mesure de tension amplifiée

(Vo), de sorte à maintenir l'amplitude constante, et
- estimer une grandeur représentative du flux d'air s'écoulant dans le canal de passage d'air (Ca, Cb, Cc) associé au capteur de mesure de flux d'air (4a, 4b, 4c) alimenté à partir de la mesure de tension amplifiée (Vo).

**2.** Appareil de mesure (1) selon la revendication 1, dans lequel l'amplitude de signal est calculée selon un cycle comprenant une période, la période dépendant de l'estimée de la grandeur représentative.

**3.** Appareil de mesure (1) selon l'une des revendications 1 et 2, dans lequel chaque circuit de mesure comprend en outre une source de courant (63) configurée pour délivrer un courant électrique de sorte à alimenter le filament métallique (41a, 41b, 41c) du capteur de mesure de flux d'air (4a, 4b, 4c), le courant électrique délivré ayant une intensité constante inférieure à 100mA.

**4.** Appareil de mesure (1) selon la revendication 3, dans lequel le microcontrôleur (61) est en outre configuré pour modifier une intensité du courant électrique délivré à partir de la mesure de tension amplifiée (Vo).

**5.** Appareil de mesure (1) selon l'une quelconque des revendications 1 à 4, dans lequel le microcontrôleur (61) est en outre configuré pour commander un préchauffage du filament métallique (41) d'au moins un capteur de mesure de flux d'air (4a, 4b, 4c) pendant une durée prédéterminée avant de mesurer la tension amplifiée (Vo).

**6.** Appareil de mesure (1) selon l'une quelconque des revendications 1 à 5, dans lequel la sonde (2) comprend en outre un capteur de température (66) configuré pour fournir une mesure de température au microcontrôleur (61).

**7.** Appareil de mesure (1) selon l'une quelconque des revendications 1 à 6, dans lequel le microcontrôleur (61) comprend en outre une horloge configurée pour mesurer une durée d'alimentation du filament métallique (41) du capteur de mesure de flux d'air (4a, 4b, 4c) alimenté, et dans lequel le microcontrôleur (61) est configuré pour émettre un signal de maintenance lorsque la durée d'alimentation mesurée dépasse une durée maximale d'utilisation.

**8.** Appareil de mesure (1) selon l'une quelconque des revendications 1 à 7, dans lequel les capteurs de mesure de flux d'air (4a, 4b, 4c) sont assemblés sur un circuit imprimé (46) modulaire connecté à la carte électronique (6) de manière amovible.

9.  Appareil de mesure (1) selon l'une quelconque des revendications 1 à 8, dans lequel au moins un capteur de mesure de flux d'air (4a, 4b, 4c) comprend un substrat (49), de préférence en céramique.

10. Appareil de mesure (1) selon la revendication 9, dans lequel :

    le filament métallique (41) comprend une pluralité de pistes résistives (41a, 41b, 41c, 41d) intégrées dans le substrat (49), ou
    le filament métallique (41) est formé par impression ou revêtement d'un matériau métallique électriquement conducteur sur le substrat (49), le matériau métallique électriquement conducteur étant de préférence du platine.

11. Appareil de mesure (1) selon la revendication 9 ou 10, dans lequel le substrat (49) et/ou le filament métallique (41) sont recouverts par une couche de protection.

12. Appareil de mesure (1) selon l'une quelconque des revendications 9 à 11, dans lequel le substrat (49) est une plaque formant une grille.

13. Appareil de mesure (1) selon la revendication 12, dans lequel le substrat (49) est souple, de sorte que le substrat (49) est propre à se déformer élastiquement lorsque le flux d'air s'écoule à travers le substrat (49) et autour du filament métallique (41).

14. Procédé de commande d'un circuit de mesure d'un appareil de mesure (1) selon l'une des revendications 1 à 13, mis en œuvre par un microcontrôleur (61), le circuit de mesure comprenant un amplificateur (62) configuré pour fournir en sortie une tension amplifiée (Vo) à partir d'une tension de référence (Vref) et d'une tension aux bornes d'un filament métallique (41) d'un capteur actif (4), le capteur actif (4) étant agencé dans un canal de passage d'air (Ca, Cb, Cc), le procédé de commande comprenant les étapes de :

    - mesure d'une tension amplifiée (Vo) en sortie de l'amplificateur (62),
    - calcul d'une amplitude de signal à partir de la mesure de tension amplifiée (Vo),
    - modification de la tension de référence (Vref) en fonction de la mesure de tension amplifiée (Vo), de sorte à maintenir l'amplitude de signal constante,
    - estimation d'une grandeur représentative d'un flux d'air s'écoulant dans le canal de passage d'air (Ca, Cb, Cc) à partir de la mesure de tension amplifiée (Vo).

15. Procédé d'assemblage d'un appareil de mesure (1) selon l'une quelconque des revendications 1 à 8, comprenant les étapes suivantes :

    - assemblage (Sa1) d'une pluralité d'ampoules à incandescence sur un circuit imprimé (46), chaque ampoule à incandescence comprenant un bulbe de protection (40) recouvrant un filament métallique (41),
    - retrait (Sa2) du bulbe de protection (40) de chaque ampoule à incandescence de sorte à obtenir une pluralité de capteurs de mesures de flux d'air (4a, 4b, 4c),
    - optionnellement, pliage (Sa3) d'au moins un capteur de mesure de flux d'air (4c), et
    - intégration (Sa5) du circuit imprimé (46) portant la pluralité de capteurs de mesures de flux d'air (4a, 4b, 4c) sur une carte électronique (6) de la sonde (2).

[Fig. 1]

[Fig. 2]

[Fig. 3]

[Fig. 4]

EP 4 741 832 A1

[Fig. 5]

[Fig. 6]

EP 4 741 832 A1

[Fig. 7]

[fig. 8]

23

[Fig. 9]

[Fig. 10]

[Fig. 11]

[Fig. 12]

[Fig. 13]

[Fig. 14]

[Fig. 15]

[Fig. 16]

[Fig. 17]

[Fig.18]

[Fig. 19]

[Fig. 20]

| Europäisches Patentamt European Patent Office Office européen des brevets | **RAPPORT DE RECHERCHE EUROPEENNE** | **Numéro de la demande** EP 25 21 4081 |

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | EP 3 361 947 B1 (AEROPHONOSCOPE [FR]) 6 mai 2020 (2020-05-06) * alinéa [0035] - alinéa [0065]; figures 1-9 * ----- | 1-15 | INV. G01P5/12 A61B5/087 G01P1/00 A61B5/097 A61B5/00 |
| A | WO 95/10980 A1 (MEDTRAC TECH INC [US]) 27 avril 1995 (1995-04-27) * page 14 - page 15; figures 1-7 * ----- | 1-15 | |
| A | US 2004/199354 A1 (HEUER DANIEL A [US] ET AL) 7 octobre 2004 (2004-10-07) * alinéa [0029]; figures 1-5 * ----- | 1-15 | |

**DOMAINES TECHNIQUES RECHERCHES (IPC)**

A61B
G01P

Le présent rapport a été établi pour toutes les revendications

| Lieu de la recherche | Date d'achèvement de la recherche | Examinateur |
|---|---|---|
| La Haye | 15 janvier 2026 | Hirsch, Arthur |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................................

& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 25 21 4081

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-01-2026

| Document brevet cité au rapport de recherche | | Date de publication | Membre(s) de la famille de brevet(s) | | Date de publication |
|---|---|---|---|---|---|
| EP 3361947 | B1 | 06-05-2020 | EP | 3361947 A1 | 22-08-2018 |
| | | | WO | 2016128627 A1 | 18-08-2016 |
| WO 9510980 | A1 | 27-04-1995 | US | 5518002 A | 21-05-1996 |
| | | | WO | 9510980 A1 | 27-04-1995 |
| US 2004199354 | A1 | 07-10-2004 | CA | 2455102 A1 | 16-07-2004 |
| | | | EP | 1441206 A1 | 28-07-2004 |
| | | | EP | 2267417 A1 | 29-12-2010 |
| | | | JP | 4118819 B2 | 16-07-2008 |
| | | | JP | 2004219424 A | 05-08-2004 |
| | | | KR | 20040066047 A | 23-07-2004 |
| | | | US | 2004199354 A1 | 07-10-2004 |

EPO FORM P0460

Pour tout renseignement concernant cette annexe : voir Journal Officiel de l'Office européen des brevets, No.12/82

**RÉFÉRENCES CITÉES DANS LA DESCRIPTION**

**Documents brevets cités dans la description**

- WO 2016128627 A1